# EUROPÄISCHE PATENTANMELDUNG

(11) **EP 2 465 843 A1**
(43) Veröffentlichungstag der Anmeldung: **20.06.2012**
(21) Anmeldenummer: 10195809.8
(22) Anmeldetag: 17.12.2010
(51) Int. Cl.: C07C 209/24, C07C 211/09, C08G 59/00

(54) **Polyamine mit sekundären aliphatischen Aminogruppen**

(71) Anmelder: Sika Technology AG, 6340 Baar (CH)
(72) Erfinder: Bütikofer, Pierre-André, 8304 Wallisellen (CH); Burckhardt, Urs, 8049 Zürich (CH)
(74) Vertreter: Sika Patent Attorneys

(57) **Zusammenfassung**

Die vorliegende Erfindung betrifft neuartige Polyamine mit sekundären Aminogruppen, ein Verfahren zu deren Herstellung, Addukte dieser Polyamine und deren Verwendungen. Die Polyamine lassen sich auf einfache Art und Weise aus leicht zugänglichen Edukten herstellen. Sie und ihre Addukte zeichnen sich insbesondere durch eine niedrige Viskosität aus und sind geeignet als Bestandteil von Polyurethan- und Polyharnstoff-Zusammensetzungen mit ausgezeichneter Verarbeitbarkeit und hoher Flexibilität, sowie als Bestandteil von Epoxidharz-Zusammensetzungen, insbesondere Beschichtungen.

## Beschreibung

### Technisches Gebiet

Die Erfindung betrifft neue Polyamine und ihre Verwendung, insbesondere als Härter in härtbaren Massen, welche insbesondere auf Epoxiden oder Isocyanaten beruhen, sowie Polyamine enthaltende härtbare Massen und ihre Verwendung, insbesondere als Beschichtungen, Beläge und Anstriche.

### Stand der Technik

Polyamine werden oft als Härter in härtbaren Massen eingesetzt, insbesondere in Epoxidharz- und in Polyurethan- oder Polyharnstoff-Zusammensetzungen. Die in Epoxidharz-Zusammensetzungen üblicherweise verwendeten Polyamine weisen einen hohen Gehalt an primären Aminogruppen auf, welche mit Kohlendioxid-Gas (CO₂) aus der Luft reagieren und dabei stabile Carbonat- und Carbamatsalze bilden können. Dies führt einerseits dazu, dass Härter auf Basis von solchen Polyaminen nicht offen an der Luft gelagert werden können, da sich sonst in den Gebinden Krusten bilden. Andererseits können mit solchen Härtern hergestellte Epoxidharz-Zusammensetzungen während und nach der Applikation CO₂ aufnehmen, was insbesondere bei Beschichtungs-Anwendungen zu Mängeln wie Trübungen, Flecken, rauer oder klebriger Oberfläche bis hin zu unvollständiger Aushärtung führen kann. Solche CO₂-bedingten Effekte werden vom Fachmann als "Blushing" bezeichnet. Zur Milderung von Blushing-Effekten und zur Verdünnung wird den Härtern oft Benzylalkohol zugesetzt, was aber wiederum Nachteile mit sich bringt, da Benzylalkohol wegen seiner Flüchtigkeit in vielen Anwendungen unerwünscht ist und grosse Mengen an Benzylalkohol die mechanischen Eigenschaften des ausgehärteten Epoxidharzes deutlich reduzieren. In Polyurethan- und Polyharnstoff-Zusammensetzungen werden als Härter Polyole und Polyamine eingesetzt. Polyole weisen dabei den Nachteil auf, dass ihre Reaktivität gegenüber Isocyanaten ähnlich hoch ist wie die Reaktivität von Isocyanaten mit Wasser, was bei hoher Luftfeuchtigkeit oder direktem Kontakt mit Wasser aufgrund des bei der Reaktion von Isocyanaten mit Wasser entstehenden CO₂ zu Blasenbildung und Schaumbildung, zu Oberflächenklebrigkeit sowie zu verminderter Haftung und Festigkeit der ausgehärteten Masse führen kann. Weiterhin müssen bei der Verwendung von Polyolen typischerweise erhebliche Mengen an Katalysatoren zugesetzt werden, um die Aushärtung bei Raumtemperatur schnell genug zu machen, was Nachteile beispielsweise bei der Toxizität oder der Beständigkeit der Zusammensetzungen mit sich bringen kann. Um diese Probleme zu vermeiden, ist es grundsätzlich attraktiv, als Härter anstelle von Polyolen Polyamine einzusetzen, da Polyamine gegenüber Isocyanaten eine wesentlich höhere Reaktivität aufweisen, damit kaum in Konkurrenz zu Wasser stehen und keine Katalysatoren benötigen. Allerdings sind die üblichen aliphatischen Polyamine gegenüber vielen Isocyanaten derart reaktiv, dass sie sich auch bei Verwendung von maschinellen Vorrichtungen kaum ausreichend in ein Polyisocyanat einmischen lassen, bevor die Mischung fest wird. Dadurch entstehen inhomogene, optisch und mechanisch unbefriedigende Produkte, und es treten Probleme mit dem Verlauf und der Substratbenetzung auf. Aromatische Polyamine sind zwar weniger reaktiv, sie sind aber stark toxisch und nicht lichtstabil, wodurch damit ausgehärtete Massen schnell vergilben.

Unter den aliphatischen Polyaminen sind solche mit vorwiegend sekundären Aminogruppen gegenüber solchen mit vorwiegend primären Aminogruppen hydrophober und weniger reaktiv; sie neigen kaum zu Blushing. Zudem sind sie im allgemeinen weniger flüchtig und weisen einen weniger starken Geruch auf. Dadurch sind sie sowohl als Härter in Epoxidharz- als auch in Polyurethan- oder Polyharnstoff-Zusammensetzungen grundsätzlich attraktiv. Viele der bekannten Polyamine mit vorwiegend sekundären Aminogruppen sind bei Raumtemperatur aber fest oder hochviskos, was ihre Verarbeitbarkeit in härtbaren Massen erschwert und teilweise den Einsatz von Lösemitteln notwendig macht, was für viele Anwendungen unerwünscht ist. Auch bezüglich ihrer Reaktivität und Verträglichkeit genügen die bekannten Polyamine mit vorwiegend sekundären Aminogruppen den Ansprüchen, die für einen erfolgreichen Einsatz in härtbaren Massen auf Basis von Epoxidharzen oder Polyisocyanaten an sie gestellt werden, oft nicht.

Da eine Vielzahl von Polyaminen mit vorwiegend primären Aminogruppen kommerziell erhältlich ist, ist es ein attraktiver Weg, Polyamine mit vorwiegend sekundären Aminogruppen ausgehend von diesen herzustellen.

Ein bekannter Weg zu sekundären Aminogruppen ist die Additionsreaktion von primären Aminogruppen mit Michael-Akzeptoren, insbesondere Acrylnitril, Acrylsäureester oder Maleinsäureester. Diese Reagentien sind preisgünstig, und die Umsetzung gelingt schon bei milden Bedingungen. Allerdings verläuft sie meist langsam und nicht vollständig und lässt damit einen Teil der Michael-Akzeptoren in unreagierter Form zurück, was deren nachträgliche Entfernung notwendig machen kann, will man eine geruchliche Störung der Produkte vermeiden. Die Verwendung von Estern als Michael-Akzeptoren kann zudem zur Bildung von unerwünschten Nebenprodukten führen, da Estergruppen mit sekundären und vor allem mit primären Aminogruppen unter Amidbildung reagieren können.

Ein weiterer bekannter Weg zu sekundären Aminogruppen ist die reduktive Alkylierung von primären Aminogruppen mit Ketonen oder Aldehyden. Auf diesem Weg hergestellte Polyamine mit vorwiegend sekundären Aminogruppen sind beispielsweise beschrieben in US 4,126,640, EP 0 438 695 und US 5,739,209. Bei der Verwendung von Ketonen entstehen dabei Polyamine mit sterisch stark gehinderten sekundären Aminogruppen, die meist wenig reaktiv sind. Die nach dem Stand der Technik verwendeten Aldehyde wiederum können zu anderen Schwierigkeiten führen. So neigen einfache aliphatische Aldehyde, wie Formaldehyd oder Acetaldehyd, durch Überalkylierung und Aminalbildung zur Bildung von unerwünschten Nebenprodukten, während stärker substituierte aliphatische Aldehyde, wie Isobutyraldehyd oder 2-Ethylhexanal, mit Epoxidharzen oft ungenügend verträgliche Produkte ergeben. Mit Benzaldehyd und anderen aromatischen Aldehyden kann die Aminierung unvollständig verlaufen, da die Benzylgruppe unter den Hydrierungsbedingungen wieder abgespalten werden kann.

Es besteht somit ein Bedarf nach bei Raumtemperatur flüssigen, niedrigviskosen Polyaminen mit sekundären aliphatischen Aminogruppen, welche eine gute Verträglichkeit mit Epoxidharzen und Polyurethan- und Polyharnstoff-Zusammensetzungen aufweisen, und welche eine nicht zu hohe Reaktivität gegenüber Isocyanaten aufweisen.

### Darstellung der Erfindung

Aufgabe der vorliegenden Erfindung ist es daher, neue Polyamine mit sekundären aliphatischen Aminogruppen zur Verfügung zu stellen, welche einfach herstellbar sind, als Härter in härtbaren Massen, insbesondere solchen auf der Basis von Epoxidharzen oder Polyisocyanaten, eingesetzt werden können und nicht mit den Nachteilen der aus dem Stand der Technik bekannten Polyamine behaftet sind.

Überraschenderweise wurde gefunden, dass Polyamine nach Anspruch 1 diese Aufgabe lösen. Es handelt sich dabei typischerweise um flüssige, relativ niedrigviskose Verbindungen mit einer geringen Flüchtigkeit und nur wenig Geruch. Sie weisen eine so geringe Reaktivität gegenüber CO₂ auf, dass sie an der Luft weder zur Bildung von Krusten noch zu Ausfällungen oder Viskositätserhöhungen neigen. Zudem sind sie durch reduktive Alkylierung von kommerziellen Polyaminen mit primären Aminogruppen mit Aldehyden, welche eine an einem tertiären C-Atom stehende Aldehydgruppe und mindestens eine sekundäre oder tertiäre Aminogruppe aufweisen, in hoher Reinheit in einem einfachen Prozess herstellbar, der keine aufwendigen Aufarbeitungsschritte erfordert.

Die Polyamine nach Anspruch 1 weisen eine gute Verträglichkeit sowohl mit Epoxidharz- als auch mit Polyurethan- und Polyharnstoff-Zusammensetzungen auf. Sie sind speziell geeignet als Härter in Epoxidharz-Beschichtungen, welche wenig Blushing-Effekte aufweisen und dabei insbesondere frei sind von Lösemitteln, Benzylalkohol und ähnlichen Verdünnern. Weiterhin sind sie speziell geeignet als Härter in Polyurethan- und Polyharnstoff-Zusammensetzungen, da sie eine moderate Reaktivität mit Isocyanatgruppen aufweisen und deshalb rasch aushärtende Beschichtungen mit einer gut handhabbaren Verarbeitungsgeschwindigkeit zugänglich machen.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Ein Gegenstand der Erfindung ist ein Polyamin der Formel (I) 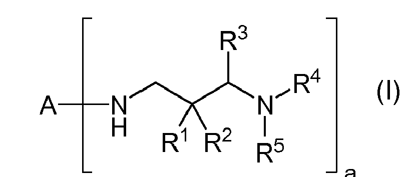 wobei
A für den Rest eines Amins nach Entfernung von a primären aliphatischen Aminogruppen steht;
a für eine ganze Zahl von 1 bis 6 steht, mit der Massgabe, dass im Fall von a = 1 der Rest A mindestens eine Reaktivgruppe ausgewählt aus der Gruppe bestehend aus primären Aminogruppen, sekundären Aminogruppen, Hydroxylgruppen und Mercaptogruppen aufweist;
R¹ und R² entweder
unabhängig voneinander jeweils für einen einwertigen Kohlenwasserstoffrest mit 1 bis 12 C-Atomen stehen,
oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
R³ für ein Wasserstoffatom oder eine Alkylgruppe oder eine Arylalkylgruppe oder eine Alkoxycarbonylgruppe steht;
und entweder
R⁴ für einen einwertigen aliphatischen, cycloaliphatischen oder arylaliphatischen Rest mit 1 bis 20 C-Atomen, welcher gegebenenfalls Heteroatome enthält, steht, und
R⁵ für ein Wasserstoffatom oder für einen einwertigen aliphatischen, cycloaliphatischen oder arylaliphatischen Rest mit 1 bis 20 C-Atomen, welcher gegebenenfalls Heteroatome enthält, steht,
oder
R⁴ und R⁵ zusammen für einen zweiwertigen aliphatischen Rest mit 3 bis 30 C-Atomen, der Teil eines, gegebenenfalls substituierten, heterocyclischen Rings mit 5 bis 8, bevorzugt 6, Ringatomen ist, wobei dieser Ring neben dem Stickstoffatom gegebenenfalls weitere Heteroatome enthält, stehen.

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.

Als "primäre Aminogruppe" wird eine NH₂-Gruppe bezeichnet, die an einen organischen Rest gebunden ist, als "sekundäre Aminogruppe" wird eine NH-Gruppe bezeichnet, die an zwei organische Reste, welche auch gemeinsam Teil eines Rings sein können, gebunden ist, und als "tertiäre Aminogruppe" wird eine Aminogruppe bezeichnet, deren Stickstoffatom ("tertiärer Amin-Stickstoff") an drei organische Reste gebunden ist, wobei zwei dieser Reste auch gemeinsam Teil eines Rings sein können.

Als "aliphatisch" wird ein Amin oder ein Isocyanat bezeichnet, deren Amino- oder Isocyanatgruppen jeweils an aliphatische, cycloaliphatische oder arylaliphatische Reste gebunden sind; entsprechend werden diese Gruppen als aliphatische Amino- oder aliphatische Isocyanatgruppen bezeichnet.

Als "aromatisch" wird ein Amin oder ein Isocyanat bezeichnet, deren Amino- oder Isocyanatgruppen jeweils an einen aromatischen Rest gebunden sind; entsprechend werden diese Gruppen als aromatische Amino- oder aromatische Isocyanatgruppen bezeichnet.

Als "primäres aliphatisches Polyamin" wird eine Substanz bezeichnet, die formal zwei oder mehr primäre aliphatische Aminogruppen pro Molekül enthält. Als "sekundäres aliphatisches Polyamin" wird eine Substanz bezeichnet, die formal zwei oder mehr sekundäre aliphatische Aminogruppen pro Molekül enthält.

Der Begriff "härtbare Masse" umfasst flüssige oder schmelzbare reaktionsfähige organische Massen und Zusammensetzungen davon, welche zumindest teilweise synthetisch hergestellt sind und für sich allein und/oder durch Luftkontakt zu Kunststoffen und Kunststoff-Zusammensetzungen aushärten können.

Der Begriff "Härter" bezeichnet Verbindungen mit mindestens zwei reaktiven Gruppen, beispielsweise in Form von primären oder sekundären Aminogruppen, Hydroxylgruppen oder Mercaptogruppen, welche als Reaktionspartner bei der Aushärtung von härtbaren Massen zu Kunststoffen dienen können.

Der Begriff "Polymer" umfasst einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Als "Raumtemperatur" wird eine Temperatur von 23 °C bezeichnet.

Der Begriff "verträglich" oder "Verträglichkeit" bezogen auf einen Härter einer härtbaren Zusammensetzung bezeichnet den Umstand, dass der Härter sich weitgehend homogen in diese Zusammensetzung einarbeiten lässt und während und nach deren Aushärtung keine auf Entmischungsphänomene zurückzuführenden Defekte, wie insbesondere Filmtrübungen oder Oberflächenstörungen in Form von Zeichnungen, Strukturen oder Ausschwimmen, verursacht.

Die fett markierten Bezeichnungen wie **PA, ALD, Y1, Y2, C, VB, RG, AD, AD1, AD2, Z1, Z2, PUP, PI, HV, S** oder dergleichen dienen lediglich für das bessere Leseverständnis und Identifizierung.

Bevorzugt steht A für einen a-wertigen Kohlenwasserstoffrest mit einem Molekulargewicht im Bereich von 28 bis 10'000 g/mol, welcher gegebenenfalls Ethergruppen, Aminogruppen, Hydroxylgruppen oder Mercaptogruppen aufweist.

Besonders bevorzugt steht A für einen a-wertigen Kohlenwasserstoffrest mit einem Molekulargewicht im Bereich von 28 bis 10'000 g/mol, welcher gegebenenfalls Ethergruppen oder primäre oder sekundäre Aminogruppen aufweist.

Bevorzugt steht a für 1 bis 3, besonders bevorzugt für 2 oder 3.

Bevorzugt stehen R¹ und R² jeweils für einen Methylrest.

Bevorzugt steht R³ für ein Wasserstoffatom.

Insbesondere stehen somit R¹ und R² jeweils für einen Methylrest und/oder R³ für ein Wasserstoffatom.

Bevorzugt stehen R⁴ für Methyl, Ethyl, Propyl, Isopropyl, Butyl, 2-Ethylhexyl, Cyclohexyl, 2-Hydroxyethyl, 2-Hydroxypropyl oder Benzyl, und R⁵ für Wasserstoff oder Methyl, Ethyl, Propyl, Isopropyl, Butyl, 2-Ethylhexyl, Cyclohexyl, 2-Hydroxyethyl, 2-Hydroxypropyl oder Benzyl.

Weiterhin bevorzugt bilden R⁴ und R⁵ zusammen - unter Einbezug des Stickstoffatoms - einen Ring, insbesondere einen Pyrrolidin-, Piperidin-, Morpholin- oder N-Alkylpiperazin-Ring, wobei dieser Ring oder die Alkylgruppe gegebenenfalls substituiert sind.

Vorteilhaft ist der Rest A frei von Alkoxysilangruppen.

Ein Polyamin der Formel (I) kann durch eine reduktiven Alkylierung mindestens eines Amins **PA** der Formel (II) mit mindestens einem Aldehyd **ALD** der Formel (III) hergestellt werden.

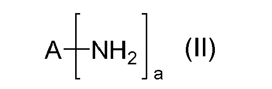

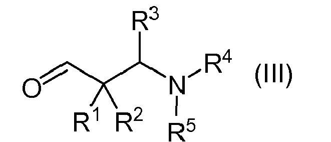

In den Formeln (II) und (III) weisen A, a, R¹, R², R³, R⁴ und R⁵ die bereits genannten Bedeutungen auf.

Der Aldehyd **ALD** kann in Bezug auf die primären Aminogruppen des Amins **PA** stöchiometrisch oder in stöchiometrischem Überschuss eingesetzt werden, wobei Polyamine der Formel (I) erhalten werden, die frei sind von primären Aminogruppen. Wenn das Amin **PA** mindestens zwei primäre Aminogruppen aufweist, kann der Aldehyd **ALD** in Bezug auf die primären Aminogruppen des Amins **PA** auch unterstöchiometrisch eingesetzt werden. Dabei entstehen Polyamine der Formel (I), die neben sekundären und gegebenenfalls tertiären Aminogruppen zusätzlich noch primäre Aminogruppen aufweisen.

Insbesondere ist ein Polyamin der Formel (I) erhältlich aus der Kondensation von mindestens einem Amin **PA** der Formel (II) mit mindestens einem Aldehyd **ALD** der Formel (III) zu einem Imin der Formel (IV), welches anschliessend hydriert wird.

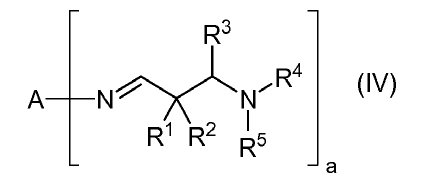

In der Formel (IV) weisen A, a, R¹, R², R³, R⁴ und R⁵ die bereits genannten Bedeutungen auf.

Die Herstellung eines Polyamins der Formel (I) kann dabei direkt aus den Reaktanden in einer Eintopfreaktion unter Hydrierung erfolgen. Das Imin der Formel (IV) kann aber auch als Zwischenprodukt isoliert und gegebenenfalls gereinigt werden, und erst anschliessend, in einer zweiten Reaktionsstufe, hydriert werden.

Das bei der Kondensation zum Imin der Formel (IV) freigesetzte Wasser muss dabei nicht entfernt werden, sondern kann während der Hydrierung in der Reaktionsmischung verbleiben. Nach der Hydrierung kann das Wasser, gegebenenfalls zusammen mit einem vorhandenen Lösemittel, beispielsweise destillativ, entfernt werden.

Die Hydrierung zur Herstellung eines Polyamins der Formel (I) kann direkt mit molekularem Wasserstoff oder indirekt durch Wasserstoff-Transfer von anderen Reagentien erfolgen. Beispiele für solche Reagentien sind Ameisensäure, wobei CO₂ freigesetzt wird (in Anlehnung an eine Leuckart-Wallach-Reaktion); Cyclohexen, welches dabei zu Benzol dehydriert wird, sowie andere Alkene wie Limonen; Organosilane; Alkalimetalle in protischen Lösemitteln; oder Hydrazin in Gegenwart eines Oxidationsmittels. Unter dem Begriff "Hydrierung" soll hier auch die Reduktion mittels Hydriden, wie beispielsweise Lithiumaluminiumhydrid, Natriumborhydrid und Natriumbis(2-methoxyethoxy)aluminiumhydrid (Vitride^{®}, Red-Al^{®}), eingeschlossen sein. Bevorzugt ist die Hydrierung mit molekularem Wasserstoff.

Der für die Hydrierung benötigte Wasserstoff wird bevorzugt bei erhöhtem Druck, insbesondere 5 bis 250 bar, und erhöhter Temperatur, insbesondere 20 bis 160 °C, in Anwesenheit eines geeigneten Katalysators verwendet. Dabei werden die Bedingungen vorteilhaft so gewählt, dass einerseits die Iminogruppen möglichst vollständig hydriert werden und andererseits möglichst keine anderen Bestandteile des Imins der Formel (IV) hydriert oder zersetzt werden.

Als Katalysatoren für die Hydrierung geeignet sind homogene Katalysatoren, wie beispielsweise Rhodium-, Ruthenium- oder Iridiumkomplexe, und insbesondere heterogene Katalysatoren, wie beispielsweise Platin, Palladium, Rhodium, Ruthenium, Osmium, Rhenium, Nickel, Kobalt oder Eisen, sowie deren Verbindungen oder Zubereitungen auf Trägermaterialien, wobei als Trägermaterialien insbesondere Bims, Kieselgur, Aluminium, Silicagel oder Aktivkohle geeignet sind. Insbesondere geeignet sind Palladium auf Kohle (Pd/C), Platin auf Kohle (Pt/C), Adams-Katalysator und Raney-Nickel. Bevorzugt sind Palladium auf Kohle und Platin auf Kohle.

Die reduktive Alkylierung wird bevorzugt in flüssiger Phase durchgeführt. Sie kann wahlweise ohne Lösemittel oder in Anwesenheit eines Lösemittels erfolgen, wobei geeignete Lösemittel unter den Reaktionsbedingungen inert sind. Insbesondere geeignet als Lösemittel sind C₁ bis C₁₀-Alkane, wie beispielsweise Hexan, Heptan oder Cyclohexan, und Alkohole, insbesondere primäre C₁ bis C₆-Alkohole wie Methanol oder Ethanol, aber auch sekundäre Alkohole wie Isopropanol und tertiäre Alkohole wie tert.-Butanol.

Die Hydrierung kann batchweise oder in einem kontinuierlichen Prozess durchgeführt werden, beispielsweise in einer kontinuierlich arbeitenden Hydrier-Apparatur. Dabei wird die zu hydrierende Substanz, gegebenenfalls in Lösung, unter Druck kontinuierlich mit Wasserstoff vermischt und durch einen geeigneten Katalysator geführt. Der Wasserstoff kann dabei mittels WasserElektrolyse kontinuierlich erzeugt werden.

In einem bevorzugten Verfahren zur Herstellung eines Polyamins der Formel (I) werden mindestens ein Amin **PA** der Formel (II), mindestens ein Aldehyd **ALD** der Formel (III) und gegebenenfalls mindestens ein geeignetes Lösemittel miteinander vermischt, die Reaktionsmischung anschliessend mit einer geeigneten Methode hydriert und danach das aus der Iminbildung freigesetzte Wasser, gegebenenfalls zusammen mit dem Lösemittel, mittels Anlegen von Vakuum entfernt.

In einem besonders bevorzugten Verfahren zur Herstellung eines Polyamins der Formel (I) werden mindestens ein Amin **PA** der Formel (II), mindestens ein Aldehyd **ALD** der Formel (III) und gegebenenfalls mindestens ein geeignetes Lösemittel miteinander vermischt, die Reaktionsmischung anschliessend in einem kontinuierlichen Verfahren hydriert und danach das aus der Iminbildung freigesetzte Wasser, gegebenenfalls zusammen mit dem Lösemittel, mittels Anlegen von Vakuum entfernt. Die Vermischung des Amins **PA** und des Aldehyds **ALD** der Formeln (II) und (III) kann dabei entweder batchweise oder ebenfalls kontinuierlich erfolgen.

Als Amin **PA** der Formel (II) geeignet sind in einer ersten Ausführungsform primäre aliphatische Polyamine, die als Härter in Epoxidgruppen oder Isocyanatgruppen aufweisenden härtbaren Massen bekannt sind, insbesondere die folgenden:
- aliphatische, cycloaliphatische oder arylaliphatische primäre Diamine, wie insbesondere Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- und 1,4-Diaminocyclohexan, Bis-(4-aminocyclohexyl)-methan (H₁₂-MDA), Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 2,5(2,6)-Bis-(aminomethyl)-bicyclo[2.2.1]heptan (NBDA), 3(4),8(9)-Bis-(aminomethyl)-tricyclo[5.2.1.0²,⁶]decan, 1,4-Diamino-2,2,6-trimethylcyclohexan (TMCDA), 1,8-Menthandiamin, 3,9-Bis-(3-aminopropyl)-2,4,8,10-tetraoxaspiro[5.5]undecan sowie 1,3- und 1,4-Xylylendiamin;
- aliphatische, cycloaliphatische oder arylaliphatische primäre Triamine wie 4-Aminomethyl-1,8-octandiamin, 1,3,5-Tris-(aminomethyl)-benzol, 1,3,5-Tris-(aminomethyl)-cyclohexan, Tris-(2-aminoethyl)-amin, Tris-(2-aminopropyl)-amin, Tris-(3-aminopropyl)-amin;
- Ethergruppen-haltige aliphatische primäre Diamine, wie insbesondere Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin und höhere Oligomere dieser Diamine, Bis-(3-aminopropyl)polytetrahydrofurane und andere Polytetrahydrofuran-diamine, sowie Polyoxyalkylen-Diamine. Letztere stellen typischerweise Produkte aus der Aminierung von Polyoxyalkylen-Diolen dar und sind beispielsweise erhältlich unter dem Namen Jeffamine^{®} (von Huntsman), unter dem Namen Polyetheramine (von BASF) oder unter dem Namen PC Amine^{®} (von Nitroil). Insbesondere geeignete Polyoxyalkylen-Diamine sind Jeffamine^{®} D-230, Jeffamine^{®} D-400, Jeffamine^{®} D-2000, Jeffamine^{®} D-4000, Jeffamine^{®} XTJ-511, Jeffamine^{®} ED-600, Jeffamine^{®} ED-900, Jeffamine^{®} ED-2003, Jeffamine^{®} XTJ-568, Jeffamine^{®} XTJ-569, Jeffamine^{®} XTJ-523, Jeffamine^{®} XTJ-536, Jeffamine^{®} XTJ-542, Jeffamine^{®} XTJ-559, Jeffamine^{®} EDR-104, Jeffamine^{®} EDR-148, Jeffamine^{®} EDR-176; Polyetheramine D 230, Polyetheramine D 400 und Polyetheramine D 2000, PC Amine^{®} DA 250, PC Amine^{®} DA 400, PC Amine^{®} DA 650 und PC Amine^{®} DA 2000;
- primäre Polyoxyalkylen-Triamine, welche typischerweise Produkte aus der Aminierung von Polyoxyalkylen-Triolen darstellen und beispielsweise erhältlich sind unter dem Namen Jeffamine^{®} (von Huntsman), unter dem Namen Polyetheramine (von BASF) oder unter dem Namen PC Amine^{®} (von Nitroil), wie insbesondere Jeffamine^{®} T-403, Jeffamine^{®} T-3000, Jeffamine^{®} T-5000, Polyetheramine T 403, Polyetheramine T 5000 und PC Amine^{®} TA 403;
- tertiäre Aminogruppen aufweisende Polyamine mit zwei primären aliphatischen Aminogruppen, wie insbesondere N,N'-Bis-(aminopropyl)-piperazin, N,N-Bis-(3-aminopropyl)methylamin, N,N-Bis-(3-aminopropyl)ethylamin, N,N-Bis-(3-aminopropyl)propylamin, N,N-Bis-(3-aminopropyl)cyclohexylamin, N,N-Bis-(3-aminopropyl)-2-ethyl-hexylamin, sowie die Produkte aus der doppelten Cyanoethylierung und nachfolgender Reduktion von Fettaminen, welche abgeleitet sind von natürlichen Fettsäuren, wie N,N-Bis-(3-aminopropyl)-dodecylamin und N,N-Bis-(3-aminopropyl)-talgalkylamin, erhältlich als Triameen^{®} Y12D und Triameen^{®} YT (von Akzo Nobel);
- tertiäre Aminogruppen aufweisende Polyamine mit drei primären aliphatischen Aminogruppen, wie insbesondere Tris-(2-aminoethyl)-amin, Tris-(2-aminopropyl)-amin und Tris-(3-aminopropyl)-amin;
- sekundäre Aminogruppen aufweisende Polyamine mit zwei primären aliphatischen Aminogruppen, wie insbesondere 3-(2-Aminoethyl)aminopropylamin, Bis-hexamethylentriamin (BHMT), Diethylentriamin (DETA), Triethylentetramin (TETA), Tetraethylenpentamin (TEPA), Pentaethylenhexamin (PEHA) und höhere Homologe linearer Polyethylenamine wie Polyethylenpolyamin mit 5 bis 7 Ethylenamin-Einheiten (sogenanntes "higher ethylenepolyamine", HEPA), Produkte aus der mehrfachen Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis-(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin und N,N'-Bis-(3-amino-1-ethylpropyl)-2-methyl-1,5-pentandiamin.

Als Amin **PA** der Formel (II) geeignet sind in einer weiteren Ausführungsform Polyamine mit nur einer primären aliphatischen Aminogruppe und mindestens einer weiteren Reaktivgruppe ausgewählt aus der Gruppe bestehend aus sekundären Aminogruppen, Hydroxylgruppen und Mercaptogruppen.

Als Polyamine mit einer primären aliphatischen Aminogruppe und mindestens einer sekundären Aminogruppe geeignet sind insbesondere N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-(2-Aminoethyl)piperazin, N-(2-Aminopropyl)piperazin, N¹-(3-(Dimethylamino)propyl)-1,3-diaminopropan (DMAPAPA), N¹-(2-(Dimethylamino)ethyl)propan-1,3-diamin, Diamine aus der Cyanoethylierung oder Cyanobutylierung und anschliessender Hydrierung von primären Monoaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin, 3-Ethylamino-1-pentylamin, 3-Butylamino-1-pentylamin, 3-Hexylamino-1-pentylamin, 3-(2-Ethylhexyl)amino-1-pentylamin, 3-Dodecylamino-1-pentylamin, 3-Cyclohexylamino-1-pentylamin, und Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, wie sie beispielsweise unter dem Handelsnamen Duomeen^{®} von Akzo Nobel erhältlich sind; von Fettaminen abgeleitete Triamine und Tetramine, wie Cocoalkyldipropylentriamin, Oleyldipropylentriamin, Talgalkyldipropylentriamin, Oleyltripropylentetramin und Talgalkyltripropylentetramin, erhältlich beispielsweise als Triameen^{®} C, Triameen^{®} OV, Triameen^{®} T, Tetrameen^{®} OV und Tetrameen^{®} T (Akzo Nobel); die Produkte aus der Michael-artigen Additionsreaktion von primären aliphatischen Diaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1

Als Polyamine mit einer primären aliphatischen Aminogruppe und mindestens einer Hydroxylgruppe geeignet sind insbesondere 2-Aminoethanol, 2-Amino-1-propanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol und höhere Homologe davon, Aminopropyldiethanolamin (APDEA), 4-(2-Aminoethyl)-2-hydroxyethylbenzol, 3-Aminomethyl-3,5,5-trimethyl-cyclohexanol, eine primäre Aminogruppe tragende Derivate von Glykolen wie Diethylenglykol, Dipropylenglykol, Dibutylenglykol und höhere Oligomere und Polymere dieser Glykole, insbesondere 2-(2-Aminoethoxy)-ethanol, 2-(2-(2-Aminoethoxy)ethoxy)ethanol, α-(2-Hydroxymethylethyl)-ω-(2-aminomethylethoxy)-poly(oxy(methyl-1 ,2-ethandiyl)); eine Hydroxylgruppe und eine primäre Aminogruppen tragende Derivate von polyalkoxylierten drei- oder höherwertigen Alkoholen; Produkte aus der einfachen Cyanoethylierung und anschliessender Hydrierung von Glykolen, insbesondere 3-(2-Hydroxyethoxy)-propylamin, 3-(2-(2-Hydroxyethoxy)-ethoxy)-propylamin und 3-(6-Hydroxyhexyloxy)-propylamin; sowie weiterhin aliphatische Polyamine einer primären und einer sekundären Aminogruppe und einer Hydroxylgruppe, wie insbesondere N-Hydroxyethyl-1,3-propandiamin, N-Hydroxypropyl-1,3-propandiamin und N3-Hydroxyethyl-1,3-pentandiamin.

Als Polyamine mit einer primären aliphatischen Aminogruppe und mindestens einer Mercaptogruppe geeignet sind insbesondere 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol und 12-Amino-1-dodecanthiol.

Als Amin **PA** der Formel (II) bevorzugt sind primäre aliphatische Polyamine.

Besonders bevorzugt ist das Amin **PA** der Formel (II) ausgewählt aus der Gruppe bestehend aus 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,12-Dodecandiamin, 1,4-Diaminocyclohexan, Bis-(4-aminocyclohexyl)-methan (H₁₂-MDA), Bis-(4-amino-3-methylcyclohexyl)-methan, 1-Amino-3-aminomethyl-3,5,5-trimethylcyclohexan (= Isophorondiamin oder IPDA), 1,3-Bis-(aminomethyl)cyclohexan, 2,5(2,6)-Bis-(aminomethyl)-bicyclo[2.2.1]heptan (NBDA), 3(4),8(9)-Bis-(aminomethyl)-tricyclo[5.2.1.0^{2,6}]decan, 1,3-Xylylendiamin, Bis-hexamethylentriamin (BHMT), Diethylentriamin (DETA), Triethylentetramin (TETA), Tetraethylenpentamin (TEPA), Pentaethylenhexamin (PEHA), Polyethylenpolyamin mit 5 bis 7 Ethylenamin-Einheiten (sogenanntes "higher ethylenepolyamine", HEPA), Dipropylentriamin (DPTA), N-(2-Aminoethyl)-1,3-propandiamin (N3-Amin), N,N'-Bis(3-aminopropyl)ethylendiamin (N4-Amin), Polyoxyalkylen-Diamine und Polyoxyalkylen-Triamine, insbesondere mit einem Molekulargewicht von 200 bis 6000 g/mol, insbesondere die kommerziellen Typen Jeffamine^{®} D-230, Jeffamine^{®} D-400, Jeffamine^{®} D-2000, Jeffamine^{®} D-4000, Jeffamine^{®} T-403, Jeffamine^{®} T-3000 und Jeffamine^{®} T-5000 (von Huntsman).

Als Aldehyd **ALD** der Formel (III) geeignet sind insbesondere die Produkte aus einer der Mannich-Reaktion analogen α-Aminoalkylierung, wie sie aus der Fachliteratur bekannt ist. Ein Aldehyd **Y1** der Formel (V), ein Aldehyd **Y2** der Formel (VI) und ein primäres oder sekundäres aliphatisches Amin **C** der Formel (VII) werden dabei unter Wasserabspaltung zu einem Aldehyd **ALD** der Formel (III) umgesetzt.

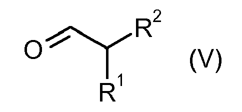

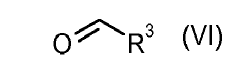

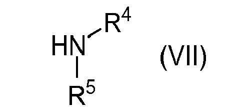

In den Formeln (V), (VI) und (VII) weisen R¹, R², R³, R⁴ und R⁵ die bereits genannten Bedeutungen auf.

Diese Umsetzung kann entweder mit den freien Reagentien **Y1, Y2** und C gemäss den Formeln (V), (VI) und (VII) geführt werden, oder die Reagentien können teilweise oder vollständig in derivatisierter Form eingesetzt werden. In einer bevorzugten Ausführungsform wird die Umsetzung mit allen Reagentien in freier Form als Eintopfreaktion geführt und der Aldehyd **ALD** nach erfolgter Umsetzung durch Destillation gereinigt. Bevorzugt werden dabei keine organischen Lösemittel eingesetzt.

Als Aldehyd **Y1** der Formel (V) geeignet sind insbesondere Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Isobutyraldehyd.

Als Aldehyd **Y2** der Formel (VI) geeignet sind insbesondere Formaldehyd, Acetaldehyd, Propionaldehyd, Butyraldehyd, Isobutyraldehyd, Phenylacetaldehyd und Glyoxylsäureester, insbesondere Glyoxylsäureethylester. Bevorzugt ist Formaldehyd.

Als Amin **C** der Formel (VII) geeignet sind insbesondere Methylamin, Dimethylamin, Ethylamin, Diethylamin, Propylamin, Dipropylamin, Isopropylamin, Diisopropylamin, Butylamin, Dibutylamin, Isobutylamin, Diisobutylamin, sek.-Butylamin, Di-sek.-Butylamin, Hexylamin, Dihexylamin, 2-Ethylhexylamin, Di-(2-ethylhexyl)amin, Octylamin, Decylamin, Dodecylamin, Ethanolamin, Diethanolamin, Isopropanolamin, Diisopropanolamin, Cyclohexylamin, Dicyclohexylamin, N-Methylbutylamin, N-Ethylbutylamin, N-Methylcyclohexylamin, N-Ethylcyclohexylamin, Bis-(2-methoxyethyl)amin, Pyrrolidin, Piperidin, Benzylamin, N-Methyl-benzylamin, N-Isopropyl-benzylamin, N-tert.Butyl-benzylamin, Dibenzylamin, Morpholin, 2,6-Dimethylmorpholin, Bis-(3-Dimethylaminopropyl)-amin, N-Methyl- oder N-Ethylpiperazin, sowie weiterhin Alkoxylate von primären Aminen wie 2-(N-Ethylamino)ethanol und 2-(N-Propylamino)ethanol. Bevorzugt sind Methylamin, Dimethylamin, Ethylamin, Diethylamin, Isopropylamin, Diisopropylamin, Butylamin, Dibutylamin, Isobutylamin, Diisobutylamin, Hexylamin, 2-Ethylhexylamin, Cyclohexylamin, N-Methylcyclohexylamin, N-Methyl-benzylamin, N-Isopropyl-benzylamin, N-tert.Butyl-benzylamin, Benzylamin, Dibenzylamin, Pyrrolidin, Piperidin, Morpholin und 2,6-Dimethylmorpholin. Besonders bevorzugt sind Methylamin, Dimethylamin und insbesondere Morpholin.

Weiterhin geeignet als Amin **C** ist Piperazin. Im Fall von Piperazin entstehen insbesondere Aldehyde **ALD** in Form von Dialdehyden, welche bei der Umsetzung mit beispielsweise primären Diaminen im Molverhältnis 1:2 zur Herstellung von Polyaminen der Formel (I) mit zwei primären und zwei sekundären Aminogruppen verwendet werden können.

Bevorzugt ist der Aldehyde **ALD** der Formel (III) ausgewählt aus der Gruppe bestehend aus 2,2-Dimethyl-3-methylamino-propanal, 2,2-Dimethyl-3-dimethylamino-propanal, 2,2-Dimethyl-3-ethylamino-propanal, 2,2-Dimethyl-3-diethylamino-propanal, 2,2-Dimethyl-3-butylamino-propanal, 2,2-Dimethyl-3-dibutylamino-propanal, 2,2-Dimethyl-3-hexylamino-propanal, 2,2-Dimethyl-3-(2-ethylhexyl)amino-propanal, 2,2-Dimethyl-3-dodecylamino-propanal, 2,2-Dimethyl-3-(N-pyrrolidino)-propanal, 2,2-Dimethyl-3-(N-piperidino)-propanal, 2,2-Dimethyl-3-(N-morpholino)-propanal, 2,2-Dimethyl-3-(N-(2,6-dimethyl)morpholino)-propanal, 2,2-Dimethyl-3-benzylamino-propanal, 2,2-Dimethyl-3-(N-benzylmethylamino)-propanal, 2,2-Dimethyl-3-(N-benzylisopropylamino)-propanal, 2,2-Dimethyl-3-cyclohexylamino-propanal, 2,2-Dimethyl-3-(N-cyclohexylmethylamino)-propanal und N,N'-Bis(2,2-dimethyl-3-oxopropyl)-piperazin.

Bei den Polyaminen der Formel (I) handelt es sich um neue Verbindungen mit überraschenden Eigenschaften. Sie sind bei Raumtemperatur typischerweise flüssig und niedrigviskos, was für viele Anwendungen ein grosser Vorteil ist. Als "niedrigviskos" werden hier Viskositäten, gemessen mit einem Kegel-Platten-Viskosimeter bei 20 °C, von unterhalb 3'000 mPa·s, insbesondere unterhalb 1'500 mPa·s, bezeichnet. Trotzdem weisen die Polyamine der Formel (I) eine geringe Flüchtigkeit und wenig Geruch auf, was für niedrigviskose Polyamine sonst selten der Fall ist. Weiterhin weisen sie im allgemeinen eine so geringe Reaktivität gegenüber CO₂ auf, dass sie - im Gegensatz zu vielen aus dem Stand der Technik bekannten Polyaminen - an der Luft weder zur Bildung von Krusten noch zu Ausfällungen oder Viskositätserhöhungen neigen. Dies ist für viele Anwendungen ein grosser Vorteil.

Ein weiterer Vorteil ist die einfache Zugänglichkeit der Polyamine der Formel (I). Sie lassen sich nämlich ausgehend von kommerziellen primären Polyaminen und den beschriebenen Aldehyden **ALD** der Formel (III) in hoher Reinheit und hoher Ausbeute in einem einfachen Prozess herstellen, der keine aufwendigen Aufarbeitungsschritte erfordert. Dies ist möglich, da die Iminbildung zwischen dem Aldehyd **ALD** und den primären Aminogruppen auch ohne aktive Wasserentfernung spontan und ohne Aminalbildung verläuft, die Hydrierung trotz sterischer Hinderung durch die tertiär substituierte Iminogruppe überraschend einfach - schon bei relativ tiefem Wasserstoffdruck und relativ niedriger Temperatur - gelingt und die entstandenen sekundären Aminogruppen der Polyamine der Formel (I) nicht an der Alkylierungsreaktion teilnehmen, es also nicht zur Überalkylierung unter Verlust an NH-Funktionalität kommen kann.

Der hohe Gehalt an Aminogruppen in Form von sekundären und gegebenenfalls tertiären Aminogruppen ermöglicht eine ganze Anzahl von vorteilhaften Verwendungen der Polyamine der Formel (I). Sie lassen sich beispielsweise überall dort einsetzen, wo niedrigviskose geruchsarme Verbindungen mit mehreren Aminogruppen erwünscht sind, zum Beispiel als Tensid, Dispergiermittel, Entschäumer, Neutralisationsmittel, Korrosionsschutzmittel, Oxidationsschutzmittel, Komplexbildner oder Katalysator, beispielsweise als Bestandteil von Reinigungsmitteln, Treibstoffen, Schmierstoffen, Asphalten, Gummiartikeln, Pharmazeutika, Pflanzenschutzmitteln, Mahlhilfsmitteln, Sequestriermitteln, Erdölförderungshilfsmitteln oder Papierchemikalien.

Besonders vorteilhaft ist die Verwendung der Polyamine der Formel (I) aber als Härter in härtbaren Massen. Dabei ist ihre Verträglichkeit und Reaktivität mit solchen Massen überraschend gut. Ohne die Erfindung dadurch einschränken zu wollen, wird vermutet, dass dies zumindest teilweise auf den Einfluss der an den Molekülenden stehenden, über die bei der reduktiven Alkylierung eingesetzten Aldehyde **ALD** der Formel (III) eingebrachten sekundären oder tertiären Aminogruppen zurückgehen könnte. Im Fall von tertiären Aminogruppen könnten diese einerseits die Verträglichkeit in härtbaren Massen erhöhen und andererseits eine katalytische Wirkung haben, und im Fall von sekundären Aminogruppen kann die Vernetzungsdichte des bei der Aushärtung der härtbaren Massen gebildeten Kunststoffes erhöht werden. Als Bestandteil von härtbaren Massen ergeben die Polyamine der Formel (I) ausgezeichnete Eigenschaften. So lassen sich mit ihnen selbstverlaufende Epoxidharz-Zusammensetzungen formulieren, welche ganz ohne oder mit nur relativ wenig an verdünnenden Zusätzen wie Lösemitteln, insbesondere Benzylalkohol, auskommen, welche bei der Aushärtung kaum zu Blushing neigen, und welche Polymerfilme von ausgezeichneter optischer und mechanischer Qualität ergeben. Weiterhin lassen sich mit ihnen rasch aushärtende Polyurethan- und Polyharnstoff-Zusammensetzungen von hoher mechanischer Qualität und ausgezeichneter Lichtstabilität erhalten, die aufgrund der vergleichsweise langen Topfzeiten über eine sehr gute Verarbeitbarkeit verfügen. Solche rasch aushärtenden Zusammensetzungen weisen den Vorteil auf, dass sie nach der Applikation sehr früh nachbearbeitet werden können.

Für die Verwendung der Polyamine der Formel (I) insbesondere geeignete härtbare Massen sind jene auf Basis von Polyepoxiden (Epoxidharze) oder von Polyisocyanaten (Polyurethane und Polyharnstoffe). Solche härtbaren Massen können beispielsweise verwendet werden als Hart- und Weichschäume, Formblöcke, Elastomere, Fasern, Faserverbundwerkstoffe (Composites), Folien oder Membranen, insbesondere als Vergussmassen, Dichtstoffe und Klebstoffe, wie beispielsweise Elektroisolationsmassen, Spachtelmassen, Fugendichtstoffe, Montageklebstoffe, Karrosserieklebstoffe, Scheibenklebstoffe, Sandwichelementklebstoffe, Halbschalenklebstoffe, z.B. für Rotorblätter von Windkraftanlagen, Kaschierklebstoffe, Laminatklebstoffe oder Verankerungsklebstoffe, sowie insbesondere als Beläge, Beschichtungen, Anstriche, Lacke, Versiegelungen, Grundierungen und Primer für Bau- und Industrieanwendungen, wie beispielsweise Bodenbeläge und Bodenbeschichtungen für Innenräume, wie Büros, Industriehallen, Turnhallen oder Kühlräume, oder im Aussenbereich für Balkone, Terrassen, Parkdecks, Brücken oder Dächer, Schutzbeschichtungen für Beton, Zement, Metalle oder Kunststoffe, beispielsweise zur Oberflächenversiegelung von Ladeflächen, Tanks, Silos, Schächten, Rohrleitungen oder Pipelines, wobei diese Beschichtungen die jeweiligen Substrate insbesondere gegen Korrosion, Abrasion, Feuchtigkeit und/oder Chemikalien schützen; sowie weiterhin als Voranstriche, Haftanstriche oder zur Hydrophobierung von Oberflächen.

Die Polyamine der Formel (I) sind also vorteilhaft verwendbar in Reinigungsmitteln, Treibstoffen, Schmierstoffen, Asphalten, Gummiartikeln, Pharmazeutika, Pflanzenschutzmitteln, Mahlhilfsmitteln, Sequestriermitteln, Erdölförderungshilfsmitteln, Papierchemikalien, Faserverbundwerkstoffen (Composites), Vergussmassen, Dichtstoffen, Klebstoffen, Belägen, Beschichtungen, Anstrichen, Lacken, Versiegelungen, Grundierungen, Primern, Schäumen, Formblöcken, Elastomeren, Fasern, Folien und Membranen.

Besonders geeignet sind die Polyamine der Formel (I) einerseits als Bestandteil von Zusammensetzungen basierend auf Epoxidharzen, insbesondere für flächige Anwendungen, beispielsweise als Bodenbeläge, Beschichtungen oder Anstriche. Die Polyamine der Formel (I) sind, wie bereits erwähnt, niedrigviskose Substanzen mit geringer Flüchtigkeit und wenig Geruch, welche spezielle sekundäre Aminogruppen aufweisen. Sie sind gut verträglich mit den üblichen kommerziellen Epoxidharzen. Durch ihre Verwendung als Härter kann der Gehalt an primären Aminogruppen von Epoxidharz-Zusammensetzungen so tief gehalten werden, dass kaum Reaktionen mit CO₂ aus der Luft auftreten. Dadurch bleiben Blushing-Effekte bei der flächigen Anwendung auch unter ungünstigen, das heisst das Blushing begünstigenden, Reaktionsbedingungen, nämlich bei tiefer Aushärtungstemperatur, beispielsweise im Bereich von 0 bis 10 °C, und hoher Luftfeuchtigkeit, weitgehend aus. Durch die Verwendung von Polyaminen der Formel (I) als Härter sind deshalb Epoxidharz-Zusammensetzungen zugänglich, welche weitgehend oder ganz frei sind von das Blushing vermindernden Zusätzen nach dem Stand der Technik, wie insbesondere Benzylalkohol, und welche in vernünftiger Zeit zu klaren, klebfreien Filmen mit schöner Oberfläche und hoher Härte aushärten.

Weiterhin besonders geeignet sind die Polyamine der Formel (I) als Bestandteil von Isocyanatgruppen aufweisenden Zusammensetzungen, insbesondere für Polyurethan- und Polyharnstoff-Beschichtungen. Bei der Aushärtung von Polyisocyanaten mit Polyaminen führt die sehr hohe Reaktivität der Aminogruppen mit Isocyanatgruppen oft zu Problemen bei der Verarbeitung. Mit den Polyaminen der Formel (I) hingegen sind Polyurethan- und Polyharnstoff-Zusammensetzungen zugänglich, welche ohne oder mit geringem Gehalt an Lösemitteln niedrigviskos sind, kaum Geruch aufweisen und über eine gut handhabbare Reaktivität verfügen, so dass gut verarbeitbare, rasch aushärtende Beschichtungen mit hoher Lichtstabilität, guten mechanischen Eigenschaften, guter Haftung zu verschiedenen Substraten und ausgezeichneten Beständigkeiten zugänglich sind.

Der Begriff "Polyurethan" umfasst sämtliche Polymere, welche nach dem sogenannten Diisocyanat-Polyadditions-Verfahren hergestellt werden. Somit weisen Polyurethane üblicherweise Urethan- oder Thiourethangruppen und insbesondere auch Harnstoffgruppen auf. Der Begriff Polyurethan schliesst aber auch solche Polymere ein, die nahezu oder gänzlich frei sind von Urethangruppen. Insbesondere sind dies sogenannte Polyharnstoffe, Polyether-Polyharnstoffe und Polyester-Polyharnstoffe, sowie weiterhin Polyether-Polyurethane, Polyester-Polyurethane, Polyisocyanurate und Polycarbodiimide.

Für die Anwendung in Isocyanatgruppen aufweisenden Zusammensetzungen besonders geeignet sind Polyamine der Formel (I), bei welchen R⁵ nicht für ein Wasserstoffatom steht, und bei welchen a für 2 oder 3, insbesondere für 2, steht, und bei welchen A frei ist von primären und sekundären Aminogruppen. Solche Polyamine der Formel (I) reagieren vergleichsweise langsam mit Isocyanatgruppen und sind gut verträglich mit Polyurethan- und Polyharnstoff-Zusammensetzungen.

Die Polyamine der Formel (I) können weiterhin zur Herstellung von Addukten verwendet werden, wobei mindestens eine Aminogruppe eines Polyamins der Formel (I) mit mindestens einer Verbindung, welche mit sekundären oder primären Aminogruppen reagieren kann, umgesetzt wird.

Ein weiterer Gegenstand der Erfindung ist ein Addukt **AD** aus der Umsetzung von mindestens einem Polyamin der Formel (I) mit mindestens einer Verbindung **VB,** die mindestens eine, bevorzugt mindestens zwei, Reaktivgruppen **RG** trägt, wobei die Reaktivgruppen **RG** ausgewählt sind aus der Gruppe bestehend aus Isocyanat-, Isothiocyanat-, Cyclocarbonat-, Epoxid-, Episulfid-, Aziridin-, Acryl-, Methacryl-, 1-Ethinylcarbonyl-, 1-Propinylcarbonyl-, Maleimid-, Citraconimid-, Vinyl-, Isopropenyl- und Allyl-Gruppen, sowie Substanzen mit verschiedenen der vorgenannten Reaktivgruppen. Bevorzugt sind Isocyanat-, Epoxid-, Acryl-, Maleimid-, Vinyl-, Isopropenyl- und Allylgruppen. Besonders bevorzugt als Reaktivgruppe **RG** ist die Epoxidgruppe und die Isocyanatgruppe.

Mindestens eine sekundäre oder primäre Aminogruppe eines Polyamins der Formel (I) reagiert dabei in einer Additionsreaktion mit mindestens einer Reaktivgruppe **RG** der Verbindung **VB** zu einem Addukt **AD.**

Die Umsetzung kann entweder so durchgeführt werden, dass die Aminogruppen des Polyamins der Formel (I) im stöchiometrischen Überschuss gegenüber den Reaktivgruppen **RG** der Verbindung **VB** vorhanden sind, wobei Addukte **AD** mit mindestens einer, bevorzugt mindestens zwei, Aminogruppen der Formel (VIII) erhältlich sind.

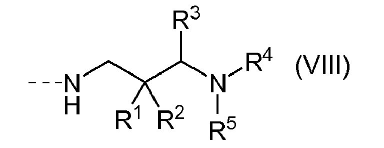

In der Formel (VIII) weisen R¹, R², R³, R⁴ und R⁵ die bereits beschriebenen Bedeutungen auf.

Die Umsetzung kann aber auch so durchgeführt werden, dass die Reaktivgruppen **RG** der Verbindung **VB** im stöchiometrischen Überschuss gegenüber den primären und sekundären Aminogruppen des Polyamins der Formel (I) vorhanden sind. Auf diese Weise sind Addukte **AD** mit mindestens einer, bevorzugt mindestens zwei, Reaktivgruppen **RG** erhältlich, wie sie vorgängig beschrieben wurden.

Die Umsetzung zwischen dem Polyamin der Formel (I) und der Verbindung **VB** zu einem Addukt **AD** erfolgt unter bekannten Bedingungen, wie sie für Reaktionen zwischen den an der jeweiligen Umsetzung beteiligten Reaktivgruppen **RG** typischerweise verwendet werden. Die Umsetzung erfolgt unter Verwendung eines Lösemittels oder bevorzugt lösemittelfrei. Gegebenenfalls können Hilfsstoffe wie beispielsweise Katalysatoren, Initiatoren oder Stabilisatoren mitverwendet werden. Die Umsetzung mit Isocyanatgruppen wird bevorzugt bei Raumtemperatur, die Umsetzung mit Epoxidgruppen bevorzugt bei erhöhter Temperatur, beispielsweise bei 40 bis 100 °C, durchgeführt.

Beispiele für geeignete Verbindungen **VB** sind
- monomere und oligomere Polyisocyanate, sowie mehr als eine Isocyanatgruppe aufweisende Umsetzungsprodukte von Polyisocyanaten mit Polyolen, wie sie im Folgenden zur Herstellung von Addukten **AD1** und **AD2** erwähnt sind;
- Polyepoxide wie Bis-(2,3-epoxycyclopentyl)ether, Polyglycidylether von mehrwertigen aliphatischen und cycloaliphatischen Alkoholen wie 1,4-Butandiol, Polypropylenglykolen und 2,2-Bis-(4-hydroxycyclohexyl)-propan; Polyglycidylether von mehrwertigen Phenolen wie Resorcinol, Bis-(4-hydroxyphenyl)-methan (Bisphenol-F), 2,2-Bis-(4-hydroxyphenyl)-propan (Bisphenol-A), 2,2-Bis-(4-hydroxy-3,5-dibromophenyl)-propan, 1,1,2,2-Tetrakis-(4-hydroxyphenyl)-ethan, Kondensationsprodukten von Phenolen mit Formaldehyd, die unter sauren Bedingungen erhalten werden, wie Phenolnovolaken und Kresolnovolaken, sowie mit diesen Alkoholen und Phenolen, oder mit Polycarbonsäuren wie beispielsweise dimeren Fettsäuren, oder einem Gemisch davon, vorverlängerte Polyglycidylether; Polyglycidylester von mehrwertigen Carbonsäuren wie Phthalsäure, Terephthalsäure, Tetrahydrophthalsäure und Hexahydrophthalsäure; N-Glycidyl-Derivate von Aminen, Amiden und heterocyclischen Stickstoffbasen wie N,N-Diglycidylanilin, N,N-Diglycidyltoluidin, N,N,O-Triglycidyl-4-aminophenol, N,N,N',N'-Tetraglycidyl-bis-(4-aminophenyl)-methan, Triglycidylcyanurat und Triglycidylisocyanurat;
- mehr als eine Acryl-, Methacryl- oder Acrylamidgruppe tragende Verbindungen wie Tris-(2-hydroxyethyl)-isocyanurat-tri(meth)acrylat, Tris-(2-hydroxyethyl)-cyanurat-tri(meth)acrylat, N,N',N"-Tris-(meth)acryloyl-perhydrotriazin;
   Acrylate und Methacrylate von aliphatischen Polyethern, Polyestern, Novolaken, Phenolen, aliphatischen oder cycloaliphatischen Alkoholen, Glykolen und Polyesterglykolen sowie mono- und polyalkoxylierten Derivaten der vorgenannten Verbindungen, beispielsweise Ethylenglykol-di(meth)acrylat, Tetraethylenglykol-di(meth)acrylat, Tripropylenglykol-di(meth)acrylat, Polyethylenglykol-di(meth)acrylat, Polypropylenglykol-di(meth)acrylat, 1,4-Butandiol-di(meth)acrylat, 1,6-Hexandiol-di(meth)acrylat, Neopentylglykol-di-(meth)acrylat, Trimethylolpropan-tri(meth)acrylat, Pentaerithritol-tetra(meth)-acrylat, Dipentaerithritol-tetra(meth)acrylat, Dipentaerithritol-penta(meth)-acrylat, Dipentaerithritol-hexa(meth)acrylat; Acryl- oder Methacryl-funktionelle Polybutadiene, Polyisoprene oder Block-copolymere davon; Addukte aus Polyepoxiden wie die oben genannten Epoxide mit Acryl- und Methacrylsäure; Polyurethan(meth)acrylate; Acrylamide wie N,N'-Methylen-bis-acrylamid;
- mehr als eine 1-Ethinylcarbonyl- oder 1-Propinylcarbonylgruppe tragende Verbindungen;
- mehr als eine Maleimid- oder Citraconimidgruppe tragende Verbindungen;
- mehr als eine Vinyl- und/oder Isopropenylgruppe tragende Vebindungen;
- mehr als eine Allylgruppe tragende Verbindungen;
- sowie heterofunktionelle, das heisst mindestens zwei verschiedene der vorgenannten Reaktivgruppen tragende, Verbindungen,

Als Verbindung **VB** besonders geeignet sind einerseits monomere und oligomere Polyisocyanate sowie mehr als eine Isocyanatgruppe aufweisende Umsetzungsprodukte von Polyisocyanaten mit Polyolen, und andererseits Polyepoxide.

Die Addukte **AD** sind für die gleichen Verwendungen geeignet wie die Polyamine der Formel (I).

Insbesondere solche Addukte **AD,** welche Aminogruppen der Formel (VIII) aufweisen, sind für dieselben Verwendungen geeignet wie die Polyamine der Formel (I), insbesondere als Härter in härtbaren Massen. Ebenso sind aber auch Addukte **AD,** welche die beschriebenen Reaktivgruppen **RG** aufweisen, geeignet als Bestandteil von härtbaren Massen.

Unter den beschriebenen Addukten **AD** besonders interessant sind in einer Ausführungsform mindestens zwei Aminogruppen der Formel (VIII) aufweisende Addukte, im Folgenden Addukte **AD1** genannt. Bevorzugte Addukte **AD1** sind abgeleitet von Polyisocyanaten als Verbindung **VB.**

Unter den beschriebenen Addukten **AD** besonders interessant sind in einer weiteren Ausführungsform mindestens zwei Isocyanatgruppen aufweisende Addukte **AD2,** zu deren Herstellung als Verbindung **VB** ein Polyisocyanat verwendet wurde.

Als Polyisocyanat zur Herstellung der Addukte **AD2** und der bevorzugten Addukte **AD1** geeignet sind insbesondere mono- und/oder oligomere aliphatische, cycloaliphatische, arylaliphatische oder aromatische Polyisocyanate wie 1,4-Tetramethylendiisocyanat, 1,6-Hexamethylendiisocyanat (HDI), 2-Methylpentamethylen-1,5-diisocyanat, 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und -1,4-diisocyanat und beliebige Gemische dieser Isomeren, 1-Methyl-2,4- und -2,6-diisocyanatocyclohexan und beliebige Gemische dieser Isomeren (HTDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI), 1,4-Diisocyanato-2,2,6-trimethylcyclohexan (TMCDI), 1,3-und 1,4-Bis-(isocyanatomethyl)-cyclohexan, m- und p-Xylylendiisocyanat (mund p-XDI), 1,3,5-Tris-(isocyanatomethyl)-benzol, m- und p-Tetramethyl-1,3-und -1,4-xylylendiisocyanat (m- und p-TMXDI), Bis-(1-Isocyanato-1-methylethyl)-naphthalin, Dimer- und Trimerfettsäureisocyanate wie 3,6-Bis-(9-isocyanatononyl)-4,5-di-(1-heptenyl)-cyclohexen (Dimeryldiisocyanat), α,α,α',α',α",α"-Hexamethyl-1,3,5-mesitylentriisocyanat, 2,4- und 2,6-Toluylen-diisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), Tris-(4-isocyanatophenyl)-methan, Tris-(4-isocyanatophenyl)-thiophosphat; Oligomere dieser Isocyanate enthaltend Uretdion-, Isocyanurat- oder Iminooxadiazindiongruppen, modifizierte Polyisocyanate enthaltend Ester-, Harnstoff-, Urethan-, Biuret-, Allophanat-, Carbodiimid-, Uretonimin- oder Oxadiazintriongruppen; sowie Isocyanat-haltige Polyurethanpolymere, das heisst mehr als eine Isocyanatgruppe aufweisende Umsetzungsprodukte von Polyisocyanaten mit zwei- oder mehr Hydroxylgruppen aufweisenden Substanzen (so genannten "Polyolen"), wie beispielsweise zwei- oder mehrwertigen Alkoholen, Glykolen oder Aminoalkoholen, polyhydroxyfunktionellen Polyethern, Polyestern, Polyacrylaten, Polycarbonaten oder Polykohlenwasserstoffen, insbesondere Polyethern.

Die Addukte **AD1** sind insbesondere geeignet als Härter in Isocyanatgruppen aufweisenden Zusammensetzungen. Sie weisen eine gut handhabbare Viskosität auf. Dies ist vor allem für Addukte basierend auf Polyisocyanaten als Verbindung **VB** nicht selbstverständlich. Werden nämlich aus dem Stand der Technik bekannte Polyamine mit Polyisocyanaten auf die beschriebene Weise adduktiert, können sich zahlreiche Schwierigkeiten ergeben. Bei Polyaminen mit vorwiegend primären Aminogruppen ist die Reaktivität mit Polyisocyanaten üblicherweise so hoch, dass die Umsetzung unkontrolliert abläuft und trotz an sich geeigneter Stöchiometrie sehr hohe Viskositäten oder sogar Feststoffe entstehen können. Aber auch bei relativ langsamen, sekundären Polyaminen ist es oftmals schwierig, Addukte mit gut handhabbaren Viskositäten zu erhalten. Die vergleichsweise tiefe Viskosität der Addukte **AD1** ist möglicherweise auf die aus dem Aldehyd **ALD** stammenden Aminogruppen, welche an die Substituenten der gebildeten Harnstoffgruppen gebunden sind, zurückzuführen, indem diese als eine Art internes Lösemittel wirken. Die Addukte **AD1** sind als Härter mit Isocyanatgruppen aufweisenden Zusammensetzungen sehr gut verträglich. Solche Zusammensetzungen verfügen über eine vergleichsweise lange Verarbeitungszeit, welche in einem ähnlichen Bereich liegt wie jene der Zusammensetzungen mit den entsprechenden Polyaminen der Formel (I) als Härter. Durch die Verwendung der Addukte **AD1** ergibt sich zudem die Möglichkeit, den volumen- und gewichtsmässigen Anteil des Härters in der Zusammensetzung zu erhöhen, was bei einer zweikomponentigen Anwendung sehr erwünscht sein kann, um ein gewünschtes Mischungsverhältnis zwischen den beiden Komponenten zu erreichen. Weiterhin ist die Aushärtung solcher Zusammensetzungen zu einem Kunststoff noch schneller, wenn als Härter ein Addukt **AD1** anstelle eines Polyamins der Formel (I) vorhanden ist, da ein Teil der zur Aushärtung notwendigen Reaktionen bereits bei der Herstellung des Adduktes stattgefunden hat. Dadurch können solche Zusammensetzung nach der Applikation noch früher nachbearbeitet werden.

Die Addukte **AD2** sind insbesondere geeignet als Polyisocyanat zur Formulierung von Polyurethan- und Polyharnstoff-Beschichtungen. Sie weisen den grossen Vorteil auf, dass ihre Isocyanatgruppen in Anwesenheit der aus der Adduktierung hervorgegangenen Harnstoffgruppen unter Ausschluss von Feuchtigkeit lagerstabil sind, was bei der Verwendung von Polyaminen aus dem Stand der Technik zur Adduktierung oft nicht der Fall ist. Die Addukte **AD2** weisen zudem eine vergleichsweise tiefe Viskosität auf, was ihre Verwendung zur Formulierung von Polyurethan- und Polyharnstoff-Beschichtungen besonders attraktiv macht. Die Verwendung der Addukte **AD2** in Polyurethan- und Polyharnstoff-Zusammensetzungen weist ebenfalls den Vorteil auf, dass auf diese Weise ein Polyamin der Formel (I) in vollständig umgesetzter Form als Bestandteil der härtbaren Zusammensetzung eingesetzt wird, was wiederum eine sehr schnelle Aushärtung zur Folge hat und das Einstellen eines gewünschten Mischungsverhältnisses einer zweikomponentigen Zusammensetzung wesentlich erleichtert.

Polyamine der Formel (I), welche primäre Aminogruppen aufweisen, können mit Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd umgesetzt werden, wobei die entstehenden Umsetzungsprodukte einen reduzierten Gehalt an primären Aminogruppen aufweisen oder ganz frei sind von primären Aminogruppen. Es ist also beispielsweise denkbar, ein primäres aliphatisches Diamin mit einer unterstöchiometrischen Menge Aldehyd **ALD** in einer reduktiven Alkylierung wie vorgängig beschrieben umzusetzen und die verbliebenen primären Aminogruppen anschliessend teilweise oder vollständig mit einem der genannten Michaelakzeptoren umzusetzen.

Ein weiterer Gegenstand der Erfindung sind härtbare Massen enthaltend mindestens ein Polyamin der Formel (I). Bevorzugt weist eine solche härtbare Masse Epoxidgruppen und/oder Isocyanatgruppen auf.

Ein weiterer Gegenstand der Erfindung ist eine härtbare Masse in Form einer Isocyanatgruppen aufweisenden Zusammensetzung **Z1,** enthaltend
a) mindestens ein Polyisocyanat, und
b) mindestens eine Härter-Verbindung **HV,** welche mindestens zwei Reaktivgruppen, ausgewählt aus der Gruppe bestehend aus primären Aminogruppen, sekundären Aminogruppen, Hydroxylgruppen und Mercaptogruppen, aufweist;
mit der Massgabe, dass das Polyisocyanat und/oder die Härter-Verbindung **HV** eine Verbindung ausgewählt aus der Gruppe bestehend aus den Polyaminen der Formel (I), den Addukten **AD1** und den Addukten **AD2** ist.

Der Begriff "Polyisocyanat" umfasst Verbindungen mit zwei oder mehr Isocyanatgruppen, unabhängig davon, ob es sich dabei um monomere Di- oder Triisocyanate, oligomere Diisocyanate oder Isocyanatgruppen aufweisende Addukte und Polymere handelt.

Als Polyisocyanat geeignet ist in einer Ausführungsform ein Addukt **AD2,** welches mindestens zwei Isocyanatgruppen aufweist, wie vorgängig beschrieben.

Als Polyisocyanat geeignet ist in einer weiteren Ausführungsform ein Isocyanatgruppen aufweisendes Polyurethanpolymer **PUP.**

Ein geeignetes Polyurethanpolymer **PUP** ist insbesondere erhältlich aus der Umsetzung von mindestens einem Polyol mit mindestens einem Polyisocyanat. Diese Umsetzung kann dadurch erfolgen, dass das Polyol und das Polyisocyanat mit üblichen Verfahren, beispielsweise bei Temperaturen von 50 °C bis 100 °C, gegebenenfalls unter Mitverwendung geeigneter Katalysatoren, zur Reaktion gebracht werden, wobei das Polyisocyanat so dosiert ist, dass dessen Isocyanatgruppen im Verhältnis zu den Hydroxylgruppen des Polyols im stöchiometrischen Überschuss vorhanden sind. Vorteilhaft ist das Polyisocyanat so dosiert, dass ein NCO/OH-Verhältnis von 1.3 bis 25, insbesondere eines von 1.5 bis 15, eingehalten wird. Unter dem "NCO/OH-Verhältnis" wird das Verhältnis der Anzahl der eingesetzten Isocyanatgruppen zu der Anzahl der eingesetzten Hydroxylgruppen verstanden. Bevorzugt verbleibt im Polyurethanpolymer **PUP** nach der Umsetzung aller Hydroxylgruppen des Polyols ein Gehalt an freien Isocyanatgruppen von 0.5 bis 30 Gewichts-%, besonders bevorzugt von 0.5 bis 25 Gewichts-%.

Gegebenenfalls kann das Polyurethanpolymer **PUP** unter Mitverwendung von Weichmachern hergestellt werden, wobei die verwendeten Weichmacher keine gegenüber Isocyanaten reaktive Gruppen enthalten.

Als Polyole für die Herstellung eines Polyurethanpolymers **PUP** können insbesondere die folgenden handelsüblichen Polyole oder Mischungen davon eingesetzt werden:
- Polyoxyalkylenpolyole, auch Polyetherpolyole oder Oligoetherole genannt, welche Polymerisationsprodukte von Ethylenoxid, 1,2-Propylenoxid, 1,2-oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten. Besonders geeignet sind Polyoxyalkylendiole oder Polyoxyalkylentriole, insbesondere Polyoxyethylen- und Polyoxypropylendi- und -triole. Speziell geeignet sind Polyoxyalkylendiole und -triole mit einem Ungesättigtheitsgrad tiefer als 0.02 mEq/g und mit einem Molekulargewicht im Bereich von 1'000 - 30'000 g/mol, sowie Polyoxypropylendiole und -triole mit einem Molekulargewicht von 400 - 8'000 g/mol.
   Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.
   Als Polyesterpolyole insbesondere geeignet sind solche, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Caprolacton und Startern wie den vorgenannten zwei- oder dreiwertigen Alkoholen.
   Besonders geeignete Polyesterpolyole sind Polyesterdiole.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/Butadien-Copolymere, wie sie beispielsweise aus Epoxiden oder Aminoalkoholen und carboxylterminierten Acrylonitril/Butadien-Copolymeren (beispielsweise kommerziell erhältlich unter dem Namen Hypro^{®} (früher Hycar^{®}) CTBN und CTBNX und ETBN von Nanoresins AG, Deutschland, bzw. Emerald Performance Materials LLC) hergestellt werden können; sowie hydrierte polyhydroxyfunktionelle Polymere oder Copolymere von Dienen.

Diese genannten Polyole weisen bevorzugt ein mittleres Molekulargewicht von 250 - 30'000 g/mol, insbesondere von 400 - 20'000 g/mol, und weisen bevorzugt eine mittlere OH-Funktionalität im Bereich von 1.6 bis 3 auf.

Als Polyole bevorzugt sind Polyether-, Polyester-, Polycarbonat- und Polyacrylatpolyole, bevorzugt Di- und Triole. Besonders bevorzugt sind Polyetherpolyole, insbesondere Polyoxypropylen- und Polyoxypropylenpolyoxyethylenpolyole, sowie flüssige Polyesterpolyole und Polyetherpolyesterpolyole.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole, 1,1,1-Trimethylolethan, 1,1,1-Trimethylolpropan, Glycerin, Pentaerythrit, Zuckeralkohole wie Xylit, Sorbit oder Mannit, Zucker wie Saccharose, andere höherwertige Alkohole, niedrigmolekulare Alkoxylierungsprodukte der vorgenannten zwei- und mehrwertigen Alkohole, sowie Mischungen der vorgenannten Alkohole bei der Herstellung des Polyurethanpolymers **PUP** mitverwendet werden. Ebenso können kleine Mengen an Polyolen mit einer mittleren OH-Funktionalität von mehr als 3 mitverwendet werden, beispielsweise Zuckerpolyole.

Als Polyisocyanat für die Herstellung eines Isocyanatgruppen aufweisenden Polyurethanpolymers **PUP** werden aromatische oder aliphatische Polyisocyanate, insbesondere die Diisocyanate, eingesetzt.

Als aromatische Polyisocyanate eignen sich insbesondere monomere Di- oder Triisocyanate wie 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), Tris-(4-isocyanatophenyl)-methan, Tris-(4-isocyanatophenyl)-thiophosphat, sowie beliebige Mischungen der vorgenannten Isocyanate. Bevorzugt sind MDI und TDI. Im Fall von MDI ist insbesondere auch 2,4'-Diphenylmethandiisocyanat bevorzugt.

Als aliphatische Polyisocyanate eignen sich insbesondere monomere Di- oder Triisocyanate wie 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und -1,4-diisocyanat, 1-Methyl-2,4- und -2,6-diisocyanatocyclohexan und beliebige Gemische dieser Isomeren (HTDI oder H₆TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder H₁₂MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), 1,3,5-Tris-(isocyanatomethyl)-benzol, Bis-(1-Isocyanato-1-methylethyl)-naphthalin, Dimer- und Trimerfettsäureisocyanate wie 3,6-Bis-(9-isocyanatononyl)-4,5-di-(1-heptenyl)-cyclohexen (Dimeryldiisocyanat), α,α,α',α',-α",α"-Hexamethyl-1,3,5-mesitylentriisocyanat, sowie beliebige Mischungen der vorgenannten Isocyanate. Bevorzugt sind HDI und IPDI.

Als Polyisocyanat geeignet ist in einer weiteren Ausführungsform ein Polyisocyanat **PI** in der Form eines monomeren Di- oder Triisocyanates oder eines Oligomeren eines monomeren Diisocyanates oder eines Derivates eines monomeren Diisocyanates, wobei als monomeres Di- oder Triisocyanat insbesondere die vorgenannten aromatischen und aliphatischen Di- und Triisocyanate geeignet sind.

Als Polyisocyanat **PI** besonders geeignet sind Oligomere oder Derivate von monomeren Diisocyanaten, insbesondere von HDI, IPDI, TDI und MDI. Kommerziell erhältliche Typen sind insbesondere HDI-Biurete, beispielsweise als Desmodur^{®} N 100 und N 3200 (von Bayer), Tolonate^{®} HDB und HDB-LV (von Rhodia) und Duranate^{®} 24A-100 (von Asahi Kasei); HDI-Isocyanurate, beispielsweise als Desmodur^{®} N 3300, N 3600 und N 3790 BA (alle von Bayer), Tolonate^{®} HDT, HDT-LV und HDT-LV2 (von Rhodia), Duranate^{®} TPA-100 und THA-100 (von Asahi Kasei) und Coronate^{®} HX (von Nippon Polyurethane); HDI-Uretdione, beispielsweise als Desmodur^{®} N 3400 (von Bayer); HDI-Iminooxadiazindione, beispielsweise als Desmodur^{®} XP 2410 (von Bayer); HDI-Allophanate, beispielsweise als Desmodur^{®} VP LS 2102 (von Bayer); IPDI-Isocyanurate, beispielsweise in Lösung als Desmodur^{®} Z 4470 (von Bayer) oder in fester Form als Vestanat^{®} T1890/100 (von Degussa); TDI-Oligomere, beispielsweise als Desmodur^{®} IL (von Bayer); sowie gemischte Isocyanurate auf Basis TDI / HDI, zum Beispiel als Desmodur^{®} HL (von Bayer). Weiterhin besonders geeeignet sind bei Raumtemperatur flüssige Formen von MDI (sogenanntes "modifiziertes MDI"), welche Gemische von MDI mit MDI-Derivaten, wie beispielsweise MDI-Carbodiimiden bzw. MDI-Uretoniminen oder MDI-Urethanen darstellen, bekannt beispielsweise unter Handelsnamen wie Desmodur^{®} CD, Desmodur^{®} PF, Desmodur^{®} PC (alle von Bayer) und Isonate^{®} 143L (von Dow) sowie Gemische aus MDI und MDI-Homologen (polymeres MDI oder PMDI), erhältlich unter Handelsnamen wie Desmodur^{®} VL, Desmodur^{®} VL50, Desmodur^{®} VL R10, Desmodur^{®} VL R20 und Desmodur^{®} VKS 20F (alle von Bayer), Isonate^{®} M 309, Voranate^{®} M 229 und Voranate^{®} M 580 (alle von Dow) oder Lupranate^{®} M 10 R (von BASF).

Die vorgenannten oligomeren Polyisocyanate **PI** stellen in der Praxis üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid-, Uretonimin- oder Oxadiazintrion-Gruppen.

Als Polyisocyanat **PI** bevorzugt sind bei Raumtemperatur flüssige Formen von MDI, sowie die Oligomeren von HDI, IPDI und TDI, insbesondere die Isocyanurate und die Biurete.

Als Polyisocyanat weiterhin geeignet sind Mischungen aus mindestens einem Addukt **AD2** und/oder mindestens einem Polyurethanpolymer **PUP** und/oder mindestens einem Polyisocyanat **PI,** wie sie vorgängig beschrieben wurden.

Als Polyisocyanat insbesondere geeignet sind sogenannte Quasi-Prepolymere. Dabei handelt es sich entweder um ein Polyurethanpolymer **PUP,** zu dessen Herstellung mindestens ein Polyol mit einem relativ grossen Überschuss mindestens eines monomeren Diisocyanates umgesetzt wurde; oder es handelt sich um das Umsetzungsprodukt von mindestens einem Polyamin mit einem relativ grossen Überschuss mindestens eines monomeren Diisocyanates. Als Polyamine sind dafür insbesondere Polyoxyalkylen-Diamine und Polyoxyalkylen-Triamine geeignet.

Solche Quasi-Prepolymere sind kommerziell erhältlich, beispielsweise als Suprasec^{®} 1007, Suprasec^{®} 2008, Suprasec^{®} 2021, Suprasec^{®} 2029, Suprasec^{®} 2054, Suprasec^{®} 2058, Suprasec^{®} 2067, Suprasec^{®} 2234, Suprasec^{®} 2444, Suprasec^{®} 2445, Suprasec^{®} 2783, Suprasec^{®} 2980, Suprasec^{®} 2982, Suprasec^{®} 9603, Suprasec^{®} 9608, Suprasec^{®} 9616, Rubinate^{®} 1209, Rubinate^{®} 1790, Rubinate^{®} 9009, Rubinate^{®} 9271, Rubinate^{®} 9447, Rubinate^{®} 9480 und Rubinate^{®} 9495, (von Huntsman); oder als Desmodur^{®} E 23, Desmodur^{®} E 210 und Desmodur^{®} E 743 (von Bayer); oder als Echelon^{®} MP 100, Echelon^{®} MP 101, Echelon^{®} MP 102, Echelon^{®} MP 104, Echelon^{®} MP 106, Echelon^{®} MP 107, Echelon^{®} MP 108 und Echelon^{®} MC 400 (von Dow); oder als Lupranate^{®} 279, Lupranate^{®} 5060 und Lupranate^{®} 5080 (von BASF).

Quasi-Prepolymere auf Basis von MDI weisen bevorzugt einen erhöhten Anteil an 2,4'-MDI auf. Solche Quasi-Prepolymere zeichnen sich durch eine geringere Reaktivität aus als solche auf Basis von vornehmlich 4,4'-MDI.

Das Polyisocyanat kann insbesondere auch eine Mischung aus mindestens einem Polyisocyanat **PI** und mindestens einem Addukt **AD2** darstellen.

Als Härter-Verbindung **HV** geeignet sind in einer Ausführungsform die vorgängig beschriebenen Polyamine der Formel (I). Bevorzugt sind Polyamine der Formel (I), bei welchen R⁵ nicht für ein Wasserstoffatom steht, und bei welchen a für 2 oder 3, insbesondere für 2, steht, und bei welchen A frei ist von primären und sekundären Aminogruppen.

Als Härter-Verbindung **HV** geeignet sind in einer weiteren Ausführungsform die vorgängig beschriebenen Addukte **AD1.** Bevorzugte Addukte **AD1** sind abgeleitet von mindestens einem Polyisocyanat als Verbindung **VB.**

Als Härter-Verbindung **HV** geeignet sind weiterhin Polyamine und Aminoalkohole, insbesondere die folgenden:
- die vorgängig beschriebenen Amine **PA** der Formel (II);
- sekundäre aliphatische Polyamine wie insbesondere N,N'-Dibutyl-ethylendiamin; N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink^{®} 754 von Huntsman), N⁴-Cyclohexyl-2-methyl-N²-(2-methylpropyl)-2,4-pentandiamin, N,N'-Dialkyl-1,3-xylylendiamin, Bis-(4-(N-alkylamino)-cyclohexyl)-methan, N-alkylierte Polyetheramine, beispielsweise die Jeffamine^{®}-Typen SD-231, SD-401, ST-404 und SD-2001 (von Huntsman), Produkte aus der Michael-artigen Additionsreaktion von primären aliphatischen Polyaminen mit Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd, sowie weiterhin kommerzielle sekundäre aliphatische Polyamine wie Gaskamine^{®} 240 (von Mitsubishi) oder die Desmophen^{®} -Typen NH 1220, NH 1420 und NH 1520 (von Bayer);
- primäre und/oder sekundäre aromatische Polyamine, wie insbesondere mund p-Phenylendiamin, 4,4'-, 2,4' und 2,2'-Diaminodiphenylmethan, 3,3'-Dichloro-4,4'-diaminodiphenylmethan (MOCA), 2,4- und 2,6-Toluylendiamin, Mischungen von 3,5-Dimethylthio-2,4- und -2,6-toluylendiamin (erhältlich als Ethacure^{®} 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)-benzolsulfonamid, 5,5'-Methylendianthranilsäure, Dimethyl-(5,5'-methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink^{®} von Air Products), 1,2-Bis-(2-aminophenylthio)-ethan, N,N'-Dialkyl-p-phenylendiamin wie beispielsweise Unilink^{®} 4100 (von UOP), N,N'-Dialkyl-4,4'-diaminodiphenylmethan wie beispielsweise Unilink^{®} 4200 (von UOP), 2-Methylpropyl-(4-chloro-3,5-diaminobenzoat) und tert.Butyl-(4-chloro-3,5-diaminobenzoat);
- Aminoalkohole wie insbesondere Diethanolamin, Diisopropanolamin, 3-Methylamino-1,2-propandiol, 2-(Methylamino)ethanol, 2-(Ethylamino)ethanol, 2-(Butylamino)ethanol, 2-(Cyclohexylamino)ethanol, 3-Pyrrolidinol, 3-oder 4-Hydroxy-piperidin, 2-Piperidin-ethanol, 2-[2-(1-piperazyl)]ethanol, 2-[2-(1-Piperazyl)ethoxy]ethanol und N-Hydroxyethylanilin.

Als Härter-Verbindung **HV** geeignet sind weiterhin handelsübliche Polyole, insbesondere die vorgängig zur Herstellung der beschriebenen Polyurethanpolymere **PUP** erwähnten Polyole, niedrigmolekularen zwei- oder mehrwertigen Alkohole und niedrigmolekularen Alkoxylierungsprodukte dieser Alkohole.

Als Härter-Verbindung **HV** geeignet sind weiterhin Polythiole, insbesondere die als mögliche Bestandteile einer Epoxidharz-Zusammensetzung **Z2** im folgenden genannten Mercaptangruppen aufweisenden Verbindungen.

Bevorzugt enthält die Isocyanatgruppen aufweisende Zusammensetzung **Z1** mindestens eine Polyoxyalkylenverbindung, besonders bevorzugt mindestens eine Polyoxypropylenverbindung, entweder in Form eines Polyoxyalkylen-Diamins oder eines Polyoxyalkylen-Triamins - gegebenenfalls in Form eines Polyamins der Formel (I) - oder in Form eines Polyoxyalkylen-Diols oder eines Polyoxyalkylen-Triols oder in Form eines Polyisocyanates **P**, welches ein Umsetzungsprodukt aus einer der genannten Polyoxyalkylenverbindungen und einem Polyisocyanat darstellt. Solche Zusammensetzungen **Z1** verfügen im ausgehärteten Zustand über eine besonders hohe Dehnbarkeit und Elastizität.

Bevorzugt enthält die Isocyanatgruppen aufweisende Zusammensetzung **Z1** mindestens eine Verbindung, welche in Bezug auf Isocyanatgruppen, primäre Aminogruppen, sekundäre Aminogruppen, Hydroxylgruppen oder Mercaptogruppen eine Funktionalität > 2 aufweist, das heisst mehr als zwei der genannten Reaktivgruppen trägt. Mögliche Verbindungen mit einer Funktionalität > 2 sind ein Addukt **AD2**, ein Polyisocyanat **P** oder eine Härter-Verbindung **HV,** insbesondere ein Triamin oder ein Triol.

Bevorzugt liegt die mittlere Funktionalität der ersten, Isocyanatgruppen aufweisenden Komponente einer zweikomponentigen Zusammensetzung **Z1** im Bereich von 1.9 bis 3.0, besonders bevorzugt im Bereich von 2.0 bis 2.5.

Bevorzugt liegt die mittlere Funktionalität der zweiten, Isocyanatgruppen-freien Komponente einer zweikomponentigen Zusammensetzung **Z1** im Bereich von 1.9 bis 3.0, besonders bevorzugt im Bereich von 2.0 bis 2.5.

Bevorzugt weist eine der beiden Komponenten einer zweikomponentigen Zusammensetzung **Z1** eine Funktionalität von etwa 2 auf.

Die Isocyanatgruppen aufweisende Zusammensetzung **Z1** enthält gegebenenfalls mindestens einen Katalysator. Als Katalysator geeignet sind einerseits stickstoffhaltige Verbindungen, insbesondere tertiäre Amine und Amidine, wie insbesondere N-Ethyl-diisopropylamin, N,N,N',N'-Tetramethylalkylendiamine, Bis-(N,N-diethylaminoethyl)-adipat, N,N,N-Tris-(3-dimethylaminopropyl)-amin, 1,4-Diazabicyclo[2.2.2]octan (DABCO), 1,8-Diazabicyclo-[5.4.0]undec-7-en (DBU), 1,5-Diazabicyclo[4.3.0]non-5-en (DBN), N-Alkylmorpholine wie N-Methylmorpholin und N-Ethylmorpholin, N,N'-Dimethylpiperazin, Benzyldimethylamin, N,N-Dimethylcyclohexylamin, N,N,N',N",N"-Pentamethyldiethylentriamin, N,N,N',N",N"-Pentamethyldipropylentriamin, N-(3-dimethylaminopropyl)-N,N-diisopropanolamin, N'-(3-(Dimethylamino)propyl)-N,N-dimethyl-1,3-propandiamin, 1-Methyl-4-(2-dimethylaminoethyl)piperazin, 1,3,5-Tris-(3-(dimethylamino)propyl)-hexahydro-s-triazin; stickstoffaromatische Verbindungen wie insbesondere 4-Dimethylamino-pyridin, N-Methylimidazol, N-Vinylimidazol oder 1,2-Dimethylimidazol; organische Ammoniumverbindungen oder alkoxylierte tertiäre Amine.

Weiterhin als Katalysator geeignet sind Metallverbindungen, insbesondere Zinnverbindungen wie insbesondere Dibutylzinndichlorid, Dibutylzinnoxid, Dibutylzinndiacetat, Dibutylzinndilaurat, Dibutylzinndiacetylacetonat, weitere Dibutylzinndicarboxylate, Dioctylzinndicarboxylate wie insbesondere Dioctylzinndilaurat, Monobutylzinntrichlorid, Zinn(II)-octoat und Alkylzinnthioester; Bismutverbindungen wie Bismuttrioctoat und Bismuttris(neodecanoat); sowie Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium.

Weiterhin als Katalysator geeignet sind Kombinationen der genannten Verbindungen, insbesondere von Metallverbindungen und stickstoffhaltigen Verbindungen.

Gegebenenfalls enthält die Isocyanatgruppen aufweisende Zusammensetzungen **Z1** weitere Bestandteile, insbesondere in Polyurethanzusammensetzungen üblicherweise eingesetzte Hilfs- und Zusatzstoffe, beispielsweise die folgenden:
- Weichmacher, insbesondere Carbonsäureester wie Phthalate, insbesondere Dioctylphthalat, Diisononylphthalat oder Diisodecylphthalat, Adipate, insbesondere Dioctyladipat, Azelate und Sebacate, organische Phosphor- und Sulfonsäureester oder Polybutene;
- Reaktivverdünner und Vernetzer, beispielsweise natürliche Harze, Fette oder Öle wie Kolophonium, Schellack, Leinöl, Rizinusöl und Sojaöl, weiterhin latente Härter wie von Polyaminen abgeleitete Aldimine, Ketimine, Enamine und Oxazolidine;
- nicht-reaktive thermoplastische Polymere, wie beispielsweise Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere Polyethylene (PE), Polypropylene (PP), Polyisobutylene, Ethylenvinylacetat-Copolymere (EVA) und ataktische Poly-α-Olefine (APAO);
- Lösemittel, wie beispielsweise Ketone wie Aceton, Methylethylketon, Diisobutylketon, Acetylaceton, Mesityloxid, Cyclohexanon, Methylcyclohexanon, Acetate wie Ethylacetat, Propylacetat, Butylacetat, Formiate, Propionate und Malonate wie Diethylmalonat; Ether wie Dialkylether, Ketonether und Esterether, beispielsweise Diisopropylether, Diethylether, Dibutylether, Diethylenglykoldiethylether und Ethylenglykoldiethylether; aliphatische und aromatische Kohlenwasserstoffe wie Toluol, Xylol, Diisopropylnaphthalin, Heptan, Octan und Erdölfraktionen wie Naphtha, White Spirit, Petrolether und Benzin, beispielsweise Solvesso^{®}-Typen (von Exxon), halogenierte Kohlenwasserstoffe wie Methylenchlorid sowie N-alkylierte Lactame wie beispielsweise N-Methylpyrrolidon;
- anorganische und organische Füllstoffe, insbesondere gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, Russ inklusive industriell hergestellte Russe, Graphit, Metall-Pulver wie Aluminium, Kupfer, Eisen, Silber oder Stahl, PVC-Pulver oder Hohlkugeln,
- Fasern, insbesondere Glasfasern, Kohlefasern, Metallfasern, Keramikfasern oder Kunststofffasern wie Polyamidfasern oder Polyethylenfasern;
- Pigmente, beispielsweise Titandioxid oder Eisenoxide;
- Rheologie-Modifizierer, wie insbesondere Verdickungsmittel, zum Beispiel Schichtsilikate wie Bentonite, Derivate von Rizinusöl, hydriertes Rizinusöl, Polyamide, Polyurethane, Harnstoffverbindungen, pyrogene Kieselsäuren, Celluloseether und hydrophob modifizierte Polyoxyethylene;
- Trocknungsmittel, wie insbesondere Molekularsiebe, Calciumoxid, hochreaktive Isocyanate wie p-Tosylisocyanat, Orthoameisensäureester, Alkoxysilane wie Tetraethoxysilan, Organoalkoxysilane wie Vinyltrimethoxysilan, und Organoalkoxysilane, welche in α-Stellung zur Silangruppe eine funktionelle Gruppe aufweisen;
- Haftverbesserer, beispielsweise Organoalkoxysilane wie Aminosilane, Mercaptosilane, Epoxysilane, Vinylsilane, (Meth)acrylsilane, Isocyanatosilane, Carbamatosilane, Alkylsilane, S-(Alkylcarbonyl)-mercaptosilane und Aldiminosilane, sowie oligomere Formen dieser Silane, insbesondere 3-Glycidoxypropyltrimethoxysilan, 3-Aminopropyltrimethoxysilan, N-(2-Aminoethyl)-3-aminopropyl-trimethoxysilan, N-(2-Aminoethyl)-N'-[3-(trimethoxysilyl)propyl]ethylendiamin, 3-Mercaptopropyltrimethoxysilan, 3-Isocyanatopropyltrimethoxysilan, 3-Ureidopropyltrimethoxysilan, 3-Chloropropyltrimethoxysilan, Vinyltrimethoxysilan, oder die entsprechenden Organosilane mit Ethoxygruppen anstelle der Methoxygruppen;
- Stabilisatoren gegen Oxidation, Wärme, Licht und UV-Strahlung;
- flammhemmende Substanzen, wie sie im folgenden als mögliche Bestandteile einer Epoxidharz-Zusammensetzung **Z2** erwähnt sind;
- oberflächenaktive Substanzen, wie beispielsweise Netzmittel, Verlaufsmittel, Entlüftungsmittel oder Entschäumer; und
- Biozide, wie beispielsweise Algizide, Fungizide oder das Pilzwachstum hemmende Substanzen.

Bevorzugt enthält die Isocyanatgruppen aufweisende Zusammensetzung **Z1** weitere Hilfs- und Zusatzstoffe, insbesondere Füllstoffe, Pigmente und Stabilisatoren.

Bevorzugt enthält die Isocyanatgruppen aufweisende Zusammensetzung **Z1** nur einen geringen Anteil an Katalysatoren, insbesondere einen Gehalt an metallhaltigen Katalysatoren von weniger als 0.02 Gewichts-% Metall. Besonders bevorzugt ist sie im wesentlichen frei von zur Erhöhung der Aushärtungsgeschwindigkeit zugefügten Katalysatoren, insbesondere metallorganischen Verbindungen wie Dibutylzinndilaurat und ähnlichen zinnorganischen Verbindungen.

Bevorzugt enthält die Isocyanatgruppen aufweisende Zusammensetzung **Z1** nur einen geringen Anteil an Lösemitteln, besonders bevorzugt weniger als 10 Gewichts-%, insbesondere weniger als 5 Gewichts-% an Lösemitteln. Am meisten bevorzugt ist die Isocyanatgruppen aufweisende Zusammensetzung **Z1** im wesentlichen frei von Lösemitteln.

Vorteilhaft liegt die Isocyanatgruppen aufweisende Zusammensetzung **Z1** in Form einer zweikomponentigen Zusammensetzung vor.

Als "zweikomponentig" wird eine härtbare Masse bezeichnet, bei welcher die Bestandteile der härtbaren Masse in zwei voneinander getrennten Komponenten vorliegen, welche jeweils in einem eigenen Gebinde gelagert werden. Erst kurz vor oder während der Applikation der härtbaren Masse werden die beiden Komponenten miteinander vermischt, worauf die vermischte Masse, gegebenenfalls unter Mitwirkung von Feuchtigkeit, aushärtet.

Eine zweikomponentige Zusammensetzung **Z1** besteht aus einer ersten, Isocyanatgruppen aufweisenden Komponente, welche mindestens ein Polyisocyanat, gegebenenfalls in Form eines Adduktes **AD2**, enthält, und einer zweiten, Isocyanatgruppen-freien Komponente, welche mindestens eine Härter-Verbindung **HV**, gegebenenfalls in Form eines Polyamins der Formel (I) oder eines Adduktes **AD1**, enthält. Weitere Bestandteile der Zusammensetzung **Z1**, beispielsweise Katalysatoren oder die erwähnten Hilfs- und Zusatzstoffe, können als Bestandteil der ersten oder als Bestandteil der zweiten Komponente vorhanden sein. Dabei ist es vorteilhaft, darauf zu achten, dass solche weiteren Bestandteile die Lagerstabilität der jeweiligen Komponente nicht stark beeinträchtigen. Das heisst, dass diese Bestandteile während der Lagerung die zur Aushärtung führenden Reaktionen nicht in signifikantem Ausmass auslösen dürfen. Insbesondere bedeutet dies, dass Substanzen, welche als Bestandteil der ersten, Isocyanatgruppen aufweisenden Komponente verwendet werden, kein oder höchstens Spuren von Wasser enthalten sollten. Es kann sinnvoll sein, gewisse Bestandteile vor dem Einmischen in die Zusammensetzung chemisch oder physikalisch zu trocknen.

Die Herstellung der beiden Komponenten erfolgt getrennt voneinander und, zumindest für die erste, Isocyanatgruppen aufweisende Komponente, unter Ausschluss von Feuchtigkeit. Die beiden Komponenten sind getrennt voneinander lagerstabil, d.h. sie können jede in einer geeigneten Verpackung oder Anordnung, wie beispielsweise einem Fass, einem Beutel, einem Eimer, einer Kartusche oder einer Flasche, vor ihrer Anwendung während mehreren Monaten bis zu einem Jahr und länger aufbewahrt werden, ohne dass sie sich in ihren jeweiligen Eigenschaften in einem für ihren Gebrauch relevanten Ausmass verändern.

Zur Anwendung einer zweikomponentigen Zusammensetzung **Z1** werden die beiden Komponenten miteinander vermischt. Dabei wird das Mischungsverhältnis zwischen den beiden Komponenten so gewählt, dass die gegenüber Isocyanatgruppen reaktiven Gruppen in einem geeigneten Verhältnis zu den Isocyanatgruppen vorliegen. Geeigneterweise liegt das Verhältnis der Anzahl von gegenüber Isocyanatgruppen reaktiven Gruppen der zweiten Komponente gegenüber der Anzahl Isocyanatgruppen der ersten Komponente im Bereich von 0.5 bis 1.1, insbesondere 0.6 bis 1.0. In Gewichtsteilen liegt das Mischungsverhältnis zwischen der ersten und der zweiten Komponente üblicherweise im Bereich von 1:10 bis 10:1.

Das Vermischen der beiden Komponenten erfolgt mittels eines geeigneten Verfahrens; es kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung kann bei Umgebungstemperatur, welche typischerweise im Bereich von etwa -20 bis 50 °C, bevorzugt bei etwa -10 bis 30 °C, liegt, erfolgen. Die Komponenten können vor dem Vermischen aber auch aufgewärmt werden, beispielsweise auf eine Temperatur im Bereich von 30 bis 80 °C, wodurch insbesondere die Viskosität der Komponenten herabgesetzt wird. Dies ist insbesondere bei der Applikation in einem Spritzverfahren vorteilhaft.

Mit der Vermischung der beiden Komponenten beginnt die Aushärtung der Zusammensetzung **Z1**, indem in der Zusammensetzung vorhandene primäre und sekundäre Aminogruppen, Hydroxylgruppen und Mercaptogruppen mit vorhandenen Isocyanatgruppen reagieren. Überschüssige Isocyanatgruppen reagieren mit Feuchtigkeit.

Somit beschreibt die vorliegende Erfindung auch eine ausgehärtete Zusammensetzung, welche durch die Reaktion von mindestens einem Polyisocyanat mit mindestens einer Härter-Verbindung **HV** einer Isocyanatgruppen aufweisenden Zusammensetzung **Z1**, wie sie vorgängig beschrieben wurde, erhalten wird.

Die Zusammensetzungen **Z1** in Form der beschriebenen Varianten weisen verschiedene vorteilhafte und überraschende Eigenschaften auf.

Liegt die Zusammensetzung **Z1** so vor, dass sie neben mindestens einem Polyisocyanat **P** mindestens ein Polyamin der Formel (I) enthält, weist sie einen geringen Amingeruch auf, ist vergleichsweise niedrigviskos und verfügt über eine hohe Aushärtungsgeschwindigkeit bei einer für sekundäre aliphatische Polyamine vergleichsweise langen Verarbeitungszeit. Im Fall von aliphatischen Polyisocyanaten **P** ist ihre Verarbeitungszeit lange genug, dass die Komponenten batchweise vermischt und in Ruhe appliziert werden können. Dadurch kann die Zusammensetzung ohne teure und schwierig zu bedienende Applikationsgeräte eingesetzt werden und muss insbesondere nicht in einem Spritzverfahren appliziert werden, was aus toxikologischen Gründen vorteilhaft ist. Im Fall von aromatischen Polyisocyanaten **P** ist die Verarbeitungszeit naturgemäss kürzer, aber bei der Applikation in einem Spritzverfahren immer noch lange genug, um ein gutes Verfliessen der applizierten Zusammensetzung und gutes Benetzen der Substrate, auf welche die Zusammensetzung appliziert wurde, zu gewährleisten. Die Aushärtung einer solchen Zusammensetzung erfolgt sehr schnell, so dass sie bereits nach kurzer Zeit nachbearbeitet werden kann. Eine Nachbearbeitung kann beispielsweise im Fall einer Beschichtung das Begehen oder Abschleifen oder Aufbringen einer weiteren Schicht sein, im Fall einer Verklebung die Belastung der Verklebung durch ihr Eigengewicht, wodurch beispielsweise Fixierungen entfernt und die verklebten Teile bewegt werden können, oder im Fall einer gegossenen Masse die Entformung, wodurch die Form für die weitere Verwendung frei wird. Die Zusammensetzung härtet auch bei hoher Luftfeuchtigkeit ohne Schaumbildung aus. Bei flächiger Applikation entstehen qualitativ hochwertige Filme von hoher Härte und Elastizität mit guter Haftung zu verschiedenen Substraten, welche insbesondere eine ausgezeichnete Beständigkeit gegenüber Abrasion, Feuchtigkeit und Chemikalien, wie organischen oder anorganischen Säuren, Laugen oder Lösemitteln, aufweisen. Im Fall von aliphatischen Polyisocyanaten **P** verfügen die ausgehärteten Filme zudem über ausgezeichnete Lichtstabilitäten ohne Neigung zu Vergilbung.

Liegt die Zusammensetzung **Z1** so vor, dass sie neben mindestens einem Polyisocyanat **P** mindestens ein Addukt **AD1** mit einem Polyisocyanat enthält, weist sie grundsätzlich eine reduzierte Empfindlichkeit gegenüber unerwünschten Einflüssen während der Aushärtung und eine noch schnellere Aushärtung bei ungefähr gleichbleibender Verarbeitungszeit auf, da ein Teil des Polyisocyanates bereits mit einem Polyamin der Formel (I) umgesetzt und somit die Anzahl der zur Aushärtung der Zusammensetzung notwendigen Reaktionen herabgesetzt ist. Ein weiterer Vorteil liegt darin, dass die gewichts- oder volumenmässigen Anteile der beiden Komponenten der Zusammensetzung einfacher auf ein gewünschtes Mischungsverhältnis zwischen der ersten und der zweiten Komponente eingestellt werden können. Insbesondere für maschinelle Applikationen ist es oftmals notwendig, ein durch die Applikationsapparatur vorgegebenes Mischungsverhältnis einzuhalten, was nach dem Stand der Technik unter Umständen schwierig ist, insbesondere wenn die Zusammensetzung frei von Lösemitteln sein soll.

Liegt die Zusammensetzung **Z1** so vor, dass sie neben mindestens einer Härter-Verbindung **HV** mindestens ein Polyisocyanat in Form eines Addukts **AD2** enthält - ein Polyamin der Formel (**I**) also in vollständig umgesetzter Form als Bestandteil der Isocyanatgruppen aufweisenden ersten Komponente vorliegt -, ergeben sich ähnliche Vorteile bezüglich der Empfindlichkeit während der Aushärtung, der Aushärtungsgeschwindigkeit sowie der Einstellung des Mischungsverhältnisses wie oben beschrieben.

Die Zusammensetzungen **Z1** sind geeignet für eine Vielzahl von Anwendungen, insbesondere als Vergussmassen, Dichtstoffe, Klebstoffe, Beläge, Beschichtungen, Anstriche, Lacke, Versiegelungen, Grundierungen, Primer und Schäume für Bau- und Industrieanwendungen. Insbesondere sind sie geeignet für Anwendungen, welche einen geringen Gehalt an Lösemitteln aufweisen oder lösemittelfrei sein sollen, sowie für Anwendungen, bei welchen eine Beschichtung oder ein Belag maschinell appliziert weren soll, insbesondere in einem Spritzverfahren.

Weiterhin ermöglichen die beschriebenen vorteilhaften Eigenschaften der Zusammensetzungen **Z1**, dass die ausgehärteten Zusammensetzungen einen hohen Gehalt an Harnstoffgruppen aufweisen können. Solche Zusammensetzungen **Z1** werden im Stand der Technik auch Polyharnstoff-Zusammensetzungen genannt. Sie härten auch bei tiefen Temperaturen und hohen Luftfeuchtigkeiten weitgehend störungsfrei aus und weisen im ausgehärteten Zustand hohe Härten und hervorragende Beständigkeiten auf. Dadurch sind sie ganz besonders geeignet für Anwendungen, welche besonders hohe Anforderungen an die Festigkeit und Beständigkeit stellen, wie beispielsweise als Bodenbeläge und Bodenbeschichtungen für Innenräume wie Büros, Industriehallen, Turnhallen oder Kühlräume, oder im Aussenbereich für Balkone, Terrassen, Parkdecks, Brücken oder Dächer, sowie als Schutzbeschichtungen für Beton, Zement, Metalle oder Kunststoffe, beispielsweise zur Oberflächenversiegelung von Ladeflächen, Tanks, Silos, Schächten, Rohleitungen oder Pipelines, wobei diese Beschichtungen die jeweiligen Substrate insbesondere gegen Korrosion, Abrasion, Feuchtigkeit und/oder Chemikalien schützen, sowie als Oberflächenversiegelung für solche Beläge. Enthalten die Zusammensetzungen **Z1** mehrheitlich oder ausschliesslich Polyisocyanate **P** mit aliphatischen Isocyanatgruppen, so weisen die ausgehärteten Zusammensetzungen eine ausgezeichnete Lichtstabilität auf, wodurch sie unter anderem auch nach längerer Lichtexposition kaum vergilben. Solche Zusammensetzungen **Z1** sind insbesondere geeignet als Oberflächenversiegelung, Deckschicht oder Decklack von Anstrichen, Beschichtungen und/oder Belägen.

Die Applikation der beschriebenen Zusammensetzung **Z1** erfolgt auf mindestens ein Substrat **S**, wobei als Substrat **S** die Folgenden besonders geeignet sind:
- Glas, Glaskeramik, Beton, Mörtel, Backstein, Ziegel, Gips und Natursteine wie Granit oder Marmor;
- Metalle und Legierungen, wie Aluminium, Eisen, Stahl und Buntmetalle, sowie oberflächenveredelte Metalle und Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Polyester, Epoxidharze, Polyurethane (PUR), Polyoxymethylen (POM), Polyolefine (PO), Polyethylen (PE) oder Polypropylen (PP), Ethylen/Propylen-Copolymere (EPM) und Ethylen/Propylen/Dien-Terpolymere (EPDM), wobei die Kunststoffe bevorzugt mittels Plasma, Corona oder Flammen oberflächenbehandelt sein können;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- beschichtete Substrate, wie pulverbeschichtete Metalle oder Legierungen;
- Farben und Lacke, insbesondere Automobildecklacke.

Die Substrate können bei Bedarf vor dem Applizieren der Zusammensetzung **Z1** vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten oder dergleichen, wobei dabei entstehende Stäube vorteilhaft abgesaugt werden, sowie weiterhin Behandeln mit Reinigern oder Lösemitteln oder das Aufbringen eines Haftvermittlers, einer Haftvermittlerlösung oder eines Primers.

Eine Isocyanatgruppen aufweisende Zusammensetzungen **Z1** kann verwendet werden in einem Verfahren zum Beschichten. Dafür werden die Komponenten der Zusammensetzung **Z1** mit einem geeigneten Verfahren miteinander vermischt und die vermischte Zusammensetzung in einer Schichtdicke von einigen Mikrometern bis zu etwa 5 mm auf ein Substrat appliziert. Je nach Reaktivität der Zusammensetzung **Z1** sind verschiedene Verfahren zum Beschichten geeignet. Zusammensetzungen **Z1** mit einer relativ langen Verarbeitungszeit können batchweise vermischt und die vermischte Zusammensetzung sofort anschliessend mittels Roller oder Rakel auf ein Substrat in der gewünschten Schichtdicke appliziert werden. Sie können aber auch in einem Spritzverfahren appliziert werden. Zusammensetzungen **Z1** mit einer kürzeren Verarbeitungszeit werden geeigneterweise in einem Spritzverfahren appliziert, wobei sowohl ein konventionelles als auch ein Airless-Spritzverfahren möglich ist. Insbesondere geeignet sind Mehrkomponenten-Spritzeinrichtungen, mittels welchen die Komponenten der Zusammensetzung **Z1** unmittelbar vor dem Spritzen vermischt werden. Zur Verminderung der Viskosität der Zusammensetzung **Z1** kann es vorteilhaft sein, die Komponenten vor dem Vermischen zu erwärmen, beispielsweise auf eine Temperatur im Bereich von 40 bis 80 °C, und/oder mit einem geeigneten Lösemittel zu verdünnen. Die Zusammensetzung **Z1** kann als Beschichtung in einer Schicht appliziert werden, sie kann aber auch in mehreren Schichten in mehreren Arbeitsgängen appliziert werden. Es kann auch vorteilhaft sein, einen Aufbau aus mehreren Schichten mit jeweils unterschiedlicher Zusammensetzung als Beschichtung zu applizieren, wobei die Zusammensetzung entweder als unterste Schicht, als Zwischenschicht oder als oberste Schicht appliziert werden kann. Eine Zusammensetzung **Z1** kann insbesondere als Schutzbeschichtung für Beton, insbesondere auf Brücken, verwendet werden, wobei die Zusammensetzung typischerweise aufgespritzt wird.

Ein weiterer Gegenstand der Erfindung ist eine härtbare Masse in Form einer Epoxidharz-Zusammensetzung **Z2**, enthaltend
a) mindestens ein Epoxidharz und
b) mindestens ein Polyamin der Formel (I).

Eine Epoxidharz-Zusammensetzung **Z2** ist insbesondere geeignet als Beschichtung oder Belag.

Als "Epoxidgruppe" oder "Epoxygruppe" wird das Strukturelement 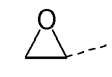 bezeichnet.

Als "Glycidylether" wird ein Ether von 2,3-Epoxy-1-propanol (Glycidol) bezeichnet.

Die Abkürzung "EEW" steht für "Epoxid-Equivalent-Gewicht".

Als Epoxidharz sind in der Epoxychemie übliche Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.

Als Epoxidharz besonders geeignet sind sogenannte Polyepoxid-Flüssigharze, im folgenden als "Flüssigharz" bezeichnet. Diese weisen eine Glasübergangstemperatur auf, welche üblicherweise unterhalb von 25 °C liegt, im Gegensatz zu den sogenannten Festharzen, welche eine Glasübergangstemperatur oberhalb von 25 °C aufweisen und sich zu bei 25 °C schüttfähigen Pulvern zerkleinern lassen.

In einer Ausführungsform handelt es sich beim Flüssigharz um ein aromatisches Polyepoxid. Dafür geeignet sind beispielsweise Flüssigharze der Formel (IX), 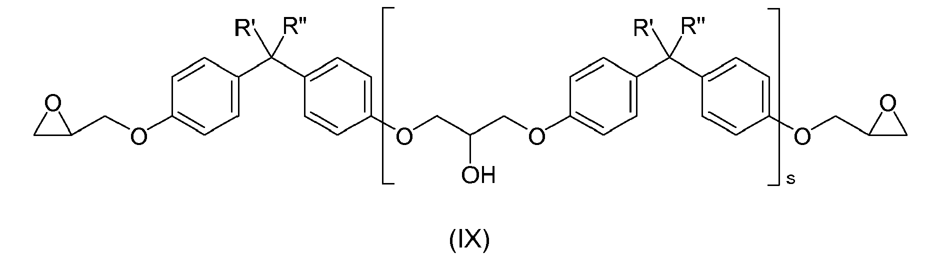 wobei R' und R" unabhängig voneinander jeweils für ein Wasserstoffatom oder für eine Methylgruppe stehen, und s im Mittel für einen Wert von 0 bis 1 steht. Bevorzugt sind solche Flüssigharze der Formel (IX), bei denen der Index s im Mittel für einen Wert von kleiner als 0.2 steht.

Bei den Flüssigharzen der Formel (IX) handelt es sich um Diglycidylether von Bisphenol-A, Bisphenol-F und Bisphenol-A/F, wobei A für Aceton und F für Formaldehyd steht, welche als Edukte zur Herstellung dieser Bisphenole dienen. Ein Bisphenol-A-Flüssigharz weist dementsprechend Methylgruppen, ein Bisphenol-F-Flüssigharz Wasserstoffatome und ein Bisphenol-A/F-Flüssigharz sowohl Methylgruppen als auch Wasserstoffatome als R' und R" in Formel (IX) auf. Im Fall von Bisphenol-F können auch Stellungsisomere vorhanden sein, insbesondere abgeleitet von 2,4'- und 2,2'-Hydroxyphenylmethan.

Weitere geeignete aromatische Flüssigharze sind die Glycidylisierungsprodukte von
- Dihydroxybenzol-Derivaten wie Resorcin, Hydrochinon und Brenzkatechin;
- weiteren Bisphenolen oder Polyphenolen wie Bis-(4-hydroxy-3-methylphenyl)-methan, 2,2-Bis-(4-hydroxy-3-methylyphenyl)-propan (Bisphenol-C), Bis-(3,5-dimethyl-4-hydroxyphenyl)-methan, 2,2-Bis-(3,5-dimethyl-4-hydroxyphenyl)-propan, 2,2-Bis-(3,5-dibromo-4-hydroxyphenyl)-propan, 2,2-Bis-(4-hydroxy-3-tert.-butylphenyl)-propan, 2,2-Bis-(4-hydroxyphenyl)-butan (Bisphenol-B), 3,3-Bis-(4-hydroxyphenyl)-pentan, 3,4-Bis-(4-hydroxyphenyl)-hexan, 4,4-Bis-(4-hydroxyphenyl)-heptan, 2,4-Bis-(4-hydroxyphenyl)-2-methylbutan, 2,4-Bis-(3,5-dimethyl-4-hydroxyphenyl)-2-methylbutan, 1,1-Bis-(4-hydroxyphenyl)-cyclohexan (Bisphenol-Z), 1,1-Bis-(4-hydroxyphenyl)-3,3,5-trimethylcyclohexan (Bisphenol-TMC), 1,1-Bis-(4-hydroxyphenyl)-1-phenylethan, 1,4-Bis[2-(4-hydroxyphenyl)-2-propyl]-benzol) (Bisphenol-P), 1,3-Bis-[2-(4-hydroxyphenyl)-2-propyl]-benzol) (Bisphenol-M), 4,4'-Dihydroxydiphenyl (DOD), 4,4'-Dihydroxybenzophenon, Bis-(2-hydroxynaphth-1-yl)-methan, Bis-(4-hydroxynaphth-1-yl)-methan 1,5-Dihydroxy-naphthalin, Tris-(4-hydroxyphenyl)-methan, 1,1,2,2-Tetrakis-(4-hydroxyphenyl)-ethan Bis-(4-hydroxyphenyl)-ether, Bis-(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendiphenyldiamin (MDA), 4,4'-Methylendiphenyldi-(N-methyl)-amin, 4,4'-[1,4-Phenylen-bis-(1-methyl-ethyliden)]-bisanilin (Bisanilin-P), 4,4'-[1,3-Phenylen-bis-(1-methyl-ethyliden)]-bisanilin (Bisanilin-M).

Als Epoxidharz eignet sich auch ein aliphatisches oder cycloaliphatisches Polyepoxid, wie beispielsweise
- ein Glycidylether eines gesättigten oder ungesättigten, verzweigten oder unverzweigten, zyklischen oder offenkettigen C₂- bis C₃₀-Diols, wie beispielsweise Ethylenglykol, Propylenglykol, Butylenglykol, Hexandiol, Octandiol, ein Polypropylenglykol, Dimethylolcyclohexan, Neopentylglykol oder Dibromo-neopentylglykol;
- ein Glycidylether eines tri- oder tetrafunktionellen, gesättigten oder ungesättigten, verzweigten oder unverzweigten, zyklischen oder offenkettigen Polyols wie Rizinusöl, Trimethylolpropan, Trimethylolethan, Pentaerythrol, Sorbit oder Glycerin, sowie alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat und Triglycidylisocyanurat, sowie Umsetzungsprodukte von Epichlorhydrin und Hydantoin.

Als Epoxidharz möglich sind auch ein Bisphenol-A-, -F- oder -A/F-Festharz, welches ähnlich aufgebaut ist wie die bereits genannten Flüssigharze der Formel (IX), aber anstelle des Index s einen Wert von 2 bis 12 aufweist, und eine Glasübergangstemperatur oberhalb von 25 °C aufweist.

Als Epoxidharz eignen sich schliesslich auch Epoxidharze aus der Oxidation von Olefinen, beispielsweise aus der Oxidation von Vinylcylohexen, Dicyclopentadien, Cyclohexadien, Cyclododecadien, Cyclododecatrien, Isopren, 1,5-Hexadien, Butadien, Polybutadien oder Divinylbenzol.

Als Epoxidharz bevorzugt sind Flüssigharze auf der Basis eines Bisphenols, insbesondere auf der Basis von Bisphenol-A, Bisphenol-F- oder Bisphenol-A/F, wie sie kommerziell beispielsweise von Dow, Huntsman und Hexion erhältlich sind, wobei diese gegebenenfalls in Kombination mit Bisphenol A-Festharz oder Bisphenol-F-Novolak-Epoxidharz vorhanden sind.

Das Epoxidharz kann einen Reaktivverdünner, insbesondere einen Epoxid-Reaktivverdünner, enthalten. Als Epoxid-Reaktivverdünner geeignet sind niedrigviskose Mono- und Polyepoxide, wie beispielsweise die Glycidylether von ein- oder mehrwertigen Phenolen und aliphatischen oder cycloaliphatischen Alkoholen, wie insbesondere die bereits genannten Polyglycidylether von Di- oder Polyolen, sowie weiterhin insbesondere Phenylglycidylether, Kresylglycidylether, p-n-Butyl-phenylglycidylether, p-tert.Butyl-phenylglycidylether, Nonylphenylglycidylether, Allylglycidylether, Butylglycidylether, Hexylglycidylether, 2-Ethylhexylglycidylether, sowie Glycidylether von natürlichen Alkoholen, wie zum Beispiel C₈- bis C₁₀-Alkylglycidylether oder C₁₂- bis C₁₄-Alkylglycidylether. Die Zugabe eines Reaktivverdünners zum Epoxidharz bewirkt eine Reduktion der Viskosität, sowie - im ausgehärteten Zustand der Epoxidharz-Zusammensetzung - eine Reduktion der Glasübergangstemperatur und der mechanischen Werte.

Die Epoxidharz-Zusammensetzung **Z2** kann zusätzlich zu mindestens einem Epoxidharz und zu mindestens einem Polyamin der Formel (I) weitere gegenüber Epoxidgruppen reaktive Verbindungen aufweisen, insbesondere weitere Polyamine und insbesondere Mercaptogruppen aufweisende Verbindungen.

Als weitere gegenüber Epoxidgruppen reaktive Verbindungen - neben den beschriebenen Polyaminen der Formel (**I**) - sind insbesondere die folgenden geeignet:
- primäre aliphatische Polyamine, Aminoalkohole und Aminothiole, wie insbesondere die bereits vorgängig beschriebenen Amine **PA** der Formel (II);
- sekundäre aliphatische Polyamine, wie sie bereits vorgängig als mögliche Härter-Verbindung **HV** einer Isocyanatgruppen aufweisenden Zusammensetzung **Z1** erwähnt wurden;
- primäre und/oder sekundäre aromatische Polyamine, wie sie bereits vorgängig als mögliche Härter-Verbindung **HV** einer Isocyanatgruppen aufweisenden Zusammensetzung **Z1** erwähnt wurden;
- Amin/Epoxid-Addukte, insbesondere Addukte aus den genannten Polyaminen mit Diepoxiden im Molverhältnis von mindestens 2/1, insbesondere im Molverhältnis von 2/1 bis 6/1, sowie Umsetzungsprodukte aus Aminen und Epichlorhydrin, insbesondere jenes von 1,3-Xyxylendiamin, kommerziell erhältlich als Gaskamine^{®} 328 (von Mitsubishi);
- Polyamidoamine, welche Umsetzungsprodukte aus einer ein- oder mehrwertigen Carbonsäure, beziehungsweise deren Ester oder Anhydride, insbesondere einer Dimerfettsäure, und einem im stöchiometrischen Überschuss eingesetzten aliphatischen, cycloaliphatischen oder aromatischen Polyamin, insbesondere einem Polyalkylenamin wie beispielsweise DETA oder TETA, darstellen, insbesondere die kommerziell erhältlichen Polyamidoamine Versamid^{®} 100, 125, 140 und 150 (von Cognis), Aradur^{®} 223, 250 und 848 (von Huntsman), Euretek^{®} 3607, Euretek^{®} 530 (von Huntsman), Beckopox^{®} EH 651, EH 654, EH 655, EH 661 und EH 663 (von Cytec);
- flüssige Mercaptan-terminierte Polysulfid-Polymere, bekannt unter dem Markennamen Thiokol^{®} (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 und LP-2; sowie weiterhin bekannt unter dem Markennamen Thioplast^{®} (von Akzo Nobel), insbesondere die Typen G 10, G 112, G 131, G 1, G 12, G 21, G 22, G 44 und G 4;
- Mercaptan-terminierte Polyoxyalkylen-Ether, erhältlich beispielsweise durch Umsetzung von Polyoxyalkylendi- und -triolen entweder mit Epichlorhydrin oder mit einem Alkylenoxid, gefolgt von Natriumhydrogensulfid;
- Mercaptan-terminierte Epoxy-Härter in Form von Polyoxyalkylen-Derivaten, bekannt unter dem Markennamen Capcure^{®} (von Cognis), insbesondere die Typen WR-8, LOF und 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri-(3-mercaptopropionat) und Glykoldi-(3-mercaptopropionat), sowie die Veresterungsprodukte von Polyoxyalkylendiolen und -triolen, ethoxyliertem Trimethylolpropan und Polyester-Diolen mit Thiocarbonsäuren wie Thioglykolsäure und 2- oder 3-Mercaptopropionsäure;
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) und Ethandithiol.

Als weitere gegenüber Epoxidgruppen reaktive Verbindungen bevorzugt sind DAMP, MPMD, C11-Neodiamin, 1,6-Hexandiamin, TMD, 1,12-Dodecandiamin, 1,3-Diaminocyclohexan, H₁₂-MDA, Bis-(4-amino-3-methylcyclohexyl)-methan, IPDA, 1,3-Xylylendiamin, N,N'-Bis(phenylethyl)-1,3-xylylendiamin (Gaskamine^{®} 240), Polyoxyalkylen-Diamine und -Triamine, insbesondere die Typen Jeffamine^{®} D-230, Jeffamine^{®} D-400 und Jeffamine^{®} T-403, sowie Amin/Epoxid-Addukte, insbesondere Gaskamine^{®} 328.

Gegebenenfalls enthält die Epoxidharz-Zusammensetzung **Z2** weitere Bestandteile, insbesondere in Epoxidharz-Zusammensetzungen üblicherweise eingesetzte Hilfs- und Zusatzstoffe, beispielsweise die folgenden:
- Lösemittel, Filmbildehilfsmittel oder Extender, wie Toluol, Xylol, Methylethylketon, 2-Ethoxyethanol, 2-Ethoxy-ethylacetat, Benzylalkohol, Ethylenglykol, Diethylenglykolbutylether, Dipropylenglykolbutylether, Ethylenglykolbutylether, Ethylenglykolphenylether, N-Methylpyrrolidon, Propylenglykolbutylether, Propylenglykolphenylether, Diphenylmethan, Diisopropylnaphthalin, Erdölfraktionen wie zum Beispiel Solvesso^{®}-Typen (von Exxon), aromatische Kohlenwasserstoffharze, insbesondere Phenolgruppen-haltige Typen, Sebacate, Phthalate, organische Phosphor- und Sulfonsäureester und Sulfonamide;
- Reaktivverdünner, beispielsweise Epoxid-Reaktivverdünner, wie sie vorgängig bereits erwähnt wurden, epoxidiertes Sojaöl oder Leinöl, Acetoacetatgruppen aufweisende Verbindungen, insbesondere acetoacetylierte Polyole, Butyrolakton, sowie weiterhin Isocyanate und Reaktivgruppenaufweisende Silikone;
- Polymere, wie beispielsweise Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere chlorsulfonierte Polyethylene und Fluor-haltige Polymere, Sulfonamid-modifizierte Melamine und gereinigte Montan-Wachse;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Dolomite, Wollastonite, Kaoline, Mica (Kalium-AluminiumSilikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Silber oder Stahl, PVC-Pulver oder Hohlkugeln,
- Fasern, insbesondere Glasfasern, Kohlefasern, Metallfasern, Keramikfasern oder Kunststofffasern wie Polyamidfasern oder Polyethylenfasern;
- Pigmente, beispielsweise Titandioxid und Eisenoxide;
- Beschleuniger, welche die Reaktion zwischen Aminogruppen und Epoxidgruppen beschleunigen, beispielsweise Säuren oder zu Säuren hydrolysierbare Verbindungen, beispielsweise organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie beispielsweise Phosphorsäure, oder Mischungen der vorgenannten Säuren und Säureester; weiterhin tertiäre Amine wie 1,4-Diazabicyclo[2.2.2]octan, Benzyldimethylamin, α-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiärer Amine, quaternäre Ammoniumsalze, wie beispielsweise Benzyltrimethylammoniumchlorid, Phenole, insbesondere Bisphenole, PhenolHarze und Mannich-Basen wie beispielsweise 2-(Dimethylaminomethyl)-phenol und 2,4,6-Tris-(dimethylaminomethyl)-phenol, Phosphite wie beispielsweise Di- und Triphenylphosphite, sowie Mercaptogruppen aufweisende Verbindungen, wie sie bereits vorgängig genannt wurden;
- Rheologie-Modifizierer, wie insbesondere Verdickungsmittel, zum Beispiel Schichtsilikate wie Bentonite, Derivate von Rizinusöl, hydriertes Rizinusöl, Polyamide, Polyurethane, Harnstoffverbindungen, pyrogene Kieselsäuren, Celluloseether und hydrophob modifizierte Polyoxyethylene;
- Haftverbesserer, beispielsweise Organoalkoxysilane wie Aminosilane, Mercaptosilane, Epoxysilane, Vinylsilane, (Meth)acrylsilane, Isocyanatosilane, Carbamatosilane, Alkylsilane, S-(Alkylcarbonyl)-mercaptosilane und Aldiminosilane, sowie oligomere Formen dieser Silane, insbesondere 3-Glycidoxypropyltrimethoxysilan, 3-Aminopropyltrimethoxysilan, N-(2-Aminoethyl)-3-aminopropyl-trimethoxysilan, N-(2-Aminoethyl)-N'-[3-(trimethoxysilyl)propyl]ethylendiamin, 3-Mercaptopropyltrimethoxysilan, 3-Isocyanatopropyltrimethoxysilan, 3-Ureidopropyltrimethoxysilan, 3-Chloropropyltrimethoxysilan, Vinyltrimethoxysilan, oder die entsprechenden Organosilane mit Ethoxygruppen anstelle der Methoxygruppen;
- Stabilisatoren gegen Oxidation, Wärme, Licht und UV-Strahlung;
- flammhemmende Substanzen, insbesondere Verbindungen wie Aluminiumhydroxid (Al(OH)₃; auch ATH für "Aluminiumtrihydrat" genannt), Magnesiumhydroxid (Mg(OH)₂; auch MDH für "Magnesiumdihydrat" genannt), Ammoniumsulfat ((NH₄)₂SO₄), Borsäure (B(OH)₃), Zinkborat, Melaminborat und Melamincyanurat; Phosphor-haltige Verbindungen wie Ammoniumphosphat ((NH₄)₃PO₄), Ammoniumpolyphosphat, Melaminphosphat, Melaminpyrophosphat, Triphenylphosphat, Diphenylkresylphosphat, Trikresylphosphat, Triethylphosphat, Tris-(2-ethylhexyl)phosphat, Trioctylphosphat, Mono-, Bis- und Tris-(isopropylphenyl)phosphat, Resorcinol-bis(diphenylphosphat), Resorcinol-diphosphat-Oligomer, Tetraphenyl-resorcinol-diphosphit, Ethylendiamin-diphosphat und Bisphenol-A-bis(diphenylphosphat); Halogen-haltige Verbindungen wie Chloroalkylphosphate, insbesondere Tris-(chloroethyl)phosphat, Tris-(chloropropyl)phosphat und Tris-(dichloroisopropyl)phosphat, polybromierte Diphenylether, insbesondere Decabromdiphenylether, polybromiertes Diphenyloxid, Tris-[3-Bromo-2,2-bis(bromo-methyl)propyl]phosphat, Tetrabromo-Bisphenol-A, Bis-(2,3-dibromopropylether) von Bisphenol A, bromierte Epoxidharze, Ethylen-bis(tetrabromophtalimid), Ethylen-bis(dibromo-norbornandicarboximid), 1,2-Bis-(tribromophenoxy)ethan, Tris-(2,3-dibromopropyl)isocyanurat, Tribromophenol, Hexabromocyclododecan, Bis-(hexachlorocyclopentadieno)cyclooctan und Chlorparaffine, sowie Kombinationen aus einer Halogen-haltigen Verbindung und Antimontrioxid (Sb₂O₃) oder Antimonpentoxid (Sb₂O₅);
- oberflächenaktive Substanzen, wie beispielsweise Netzmittel, Verlaufsmittel, Entlüftungsmittel oder Entschäumer;
- Biozide, wie beispielsweise Algizide, Fungizide oder das Pilzwachstum hemmende Substanzen.

Bevorzugt enthält die Epoxidharz-Zusammensetzung **Z2** weitere Hilfs- und Zusatzstoffe, insbesondere Netzmittel, Verlaufsmittel, Entschäumer, Stabilisatoren, Pigmente und Beschleuniger, insbesondere Salicylsäure oder 2,4,6-Tris-(dimethylaminomethyl)-phenol.

Bevorzugt enthält die Epoxidharz-Zusammensetzung **Z2** nur einen geringen Anteil an Lösemitteln, besonders bevorzugt weniger als 10 Gewichts-%, insbesondere weniger als 5 Gewichts-% an Lösemiteln. Am meisten bevorzugt ist die Zusammensetzung im Wesentlichen frei von Lösemitteln, wobei der Begriff "Lösemittel" im vorliegenden Dokument auch Stoffe wie Benzylalkohol und Alkylphenole einschliesst.

Vorteilhaft liegt die Epoxidharz-Zusammensetzung **Z2** in Form einer zweikomponentigen Zusammensetzung vor.

Eine zweikomponentige Epoxidharz-Zusammensetzung **Z2** besteht aus einer sogenannten Harz-Komponente, welche mindestens ein Epoxidharz enthält, und einer sogenannten Härter-Komponente, welche mindestens ein Polyamin der Formel (I) und gegebenenfalls weitere gegenüber Epoxidgruppen reaktive Verbindungen enthält.

Weitere Bestandteile einer zweikomponentigen Epoxidharz-Zusammensetzung **Z2** können als Bestandteil der Harz- oder der Härter-Komponente vorhanden sein.

Die Harz-Komponente und die Härter-Komponente einer zweikomponentigen Epoxidharz-Zusammensetzung **Z2** können jeweils in einer geeigneten Verpackung oder Anordnung, wie beispielsweise einem Fass, einem Hobbock, einem Beutel, einem Eimer, einer Büchse, einer Kartusche oder einer Tube, vor ihrer Anwendung während mehreren Monaten bis zu einem Jahr und länger aufbewahrt werden, ohne dass sie sich in ihren jeweiligen Eigenschaften in einem für ihren Gebrauch relevanten Ausmass verändern.

Zur Anwendung einer zweikomponentigen Epoxidharz-Zusammensetzung Z2 werden die Harz-Komponente und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen.

Geeigneterweise liegt das Verhältnis der Anzahl von gegenüber Epoxidgruppen reaktiven Gruppen der Härter-Komponente gegenüber der Anzahl Epoxidgruppen der Harz-Komponente im Bereich von 0.5 bis 1.5, insbesondere 0.8 bis 1.2.

Dem Fachmann ist bekannt, dass primäre Aminogruppen gegenüber Epoxidgruppen difunktionell sind, und eine primäre Aminogruppe somit als zwei gegenüber Epoxidgruppen reaktive Gruppen zählt.

In Gewichtsteilen liegt das Mischungsverhältnis zwischen der Harz-Komponente und der Härter-Komponente üblicherweise im Bereich von 1:10 bis 10:1.

Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche für die Applikation einer Epoxidharz-Zusammensetzung typischerweise im Bereich von etwa 0 bis 50 °C, bevorzugt bei etwa 10 bis 30 °C, liegt.

Mit der Vermischung der beiden Komponenten beginnt die Aushärtung der beschriebenen Epoxidharz-Zusammensetzung **Z2** durch chemische Reaktion. Dabei reagieren die in der vermischten Zusammensetzung vorhandenen NH-Wasserstoffe der Polyamine der Formel (I) und gegebenenfalls vorhandene weitere gegenüber Epoxidgruppen reaktive Gruppen mit Epoxidgruppen unter deren Ringöffnung (Additionsreaktion). Als Ergebnis dieser Reaktionen polymerisiert die Zusammensetzung und härtet schliesslich aus. Die Aushärtung erfolgt insbesondere bei Umgebungstemperatur, welche typischerweise im Bereich von etwa 0 bis 50 °C, bevorzugt bei etwa 10 bis 30 °C, liegt. Die Aushärtung erstreckt sich typischerweise über einige Tage bis Wochen, bis sie unter den gegebenen Bedingungen weitgehend abgeschlossen ist. Die Dauer hängt unter anderem von der Temperatur, der Reaktivität der Bestandteile und deren Stöchiometrie sowie der Gegenwart von Beschleunigern ab.

Somit beschreibt die vorliegende Erfindung auch eine ausgehärtete Zusammensetzung, welche durch die Reaktion von mindestens einem Epoxidharz mit mindestens einem Polyamin der Formel (I) einer Epoxidharz-Zusammensetzung **Z2**, wie sie vorgängig beschrieben wurde, erhalten wird.

Die Applikation der beschriebenen Epoxidharz-Zusammensetzungen **Z2** erfolgt auf mindestens ein Substrat, wobei als Substrat die bereits genannten Substrate **S** geeignet sind. Die Substrate **S** können bei Bedarf vor dem Applizieren der Epoxidharz-Zusammenetzung vorbehandelt werden, wie vorgängig bereits beschrieben.

Die in den Epoxidharz-Zusammensetzungen **Z2** enthaltenen Polyamine der Formel (I) sind, wie bereits erwähnt, typischerweise niedrigviskose Substanzen mit geringer Flüchtigkeit und wenig Geruch, welche mit den üblichen kommerziellen Epoxidharzen überraschend gut verträglich sind. Ohne die Erfindung dadurch einschränken zu wollen, wird vermutet, dass die gute Verträglichkeit zumindest teilweise auf den Einfluss der an den Molekülenden stehenden, über die bei der reduktiven Alkylierung eingesetzten Aldehyde **ALD** der Formel (III) eingebrachten Gruppierungen zurückgehen könnte.

Eine Epoxidharz-Zusammensetzung **Z2** kann in einem Verfahren zum Beschichten verwendet werden. Dafür weist die Epoxidharz-Zusammensetzung **Z2** vorteilhaft eine flüssige Konsistenz mit guten Verlaufseigenschaften auf. Sie kann dadurch insbesondere als selbstverlaufende Beschichtung auf überwiegend ebene Flächen appliziert werden, beispielsweise als Bodenbelag. Dies bedingt, dass die Zusammensetzung bei der Applikation eine niedrige Viskosität aufweist. Bevorzugt weist die Epoxidharz-Zusammensetzung **Z2** bei der Applikation - also unmittelbar nach dem Vermischen der Harz- und der Härter-Komponente - eine Viskosität, gemessen mit einem Kegel-Platten-Viskosimeter bei 20 °C, im Bereich von 100 bis 3'000 mPa·s, insbesondere im Bereich von 100 bis 2'000 mPa·s, am meisten bevorzugt im Bereich von 100 bis 1'500 mPa·s, auf. Solche für Epoxidharz-Zusammensetzungen vergleichsweise niedrige Viskositäten lassen sich üblicherweise nur durch den Einsatz von niedrigviskosen Härtern erreichen, wenn auf den Einsatz von Lösemitteln und Verdünnern verzichtet werden soll. Es ist deshalb ein wesentlicher Aspekt der Erfindung, dass es sich bei den Polyaminen der Formel (I) typischerweise um flüssige und niedrigviskose Verbindungen handelt.

Vor der Applikation werden die Harz- und die Härter-Komponente auf geeignete Weise miteinander vermischt und die vermischte Zusammensetzung innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt beispielsweise durch Aufgiessen auf das zu beschichtende Substrat. Dabei wird die vermischte Zusammensetzung im flüssigen Zustand mit Hilfe beispielsweise eines Rakels oder einer Zahntraufel gleichmässig verteilt. Zusätzlich kann die vermischte, verteilte Zusammensetzung mit einer Stachelwalze egalisiert und entlüftet werden. Es ist aber auch eine maschinelle Applikation, beispielsweise in Form einer Spritzapplikation, möglich.

Ebenfalls möglich ist der Auftrag einer vermischten zweikomponentigen Epoxidharz-Zusammensetzung **Z2** mittels Pinsel, Tuch, Schwamm oder einer Spritzpistole auf ein Substrat, insbesondere wenn die Zusammensetzung in einer dünnen Schicht von weniger als 1 mm, beispielsweise als Grundierung oder als Oberflächenversiegelung, aufgetragen werden soll. Für eine solche Anwendung enthält die Zusammensetzung **Z2** üblicherweise Lösemittel.

Bei der Aushärtung entstehen typischerweise weitgehend klare, glänzende und klebefreie Filme, welche ausgezeichnete mechanische Eigenschaften, wie hohe Härte, gute Kratzfestigkeit und Zähigkeit sowie eine gute Haftung zu verschiedensten Substraten, aufweisen. Im Gegensatz dazu härten Epoxidharz-Zusammensetzungen nach dem Stand der Technik, welche anstelle von Polyaminen der Formel (I) Härter mit vorwiegend primären Aminogruppen enthalten, oftmals zu Filmen mit Blushing-bedingten Oberflächendefekten wie Rauheit, Fleckigkeit, Trübheit und Klebrigkeit.

Durch die Verwendung der Polyamine der Formel (I) als Härter sind somit selbstverlaufende Epoxidharz-Beschichtungen zugänglich, welche ganz ohne oder mit nur relativ geringen Anteilen an verdünnenden Zusätzen wie Lösemitteln auskommen. Weiterhin kann der Gehalt an primären Aminogruppen in den Beschichtungen so tief gehalten werden, dass kaum Reaktionen mit CO₂ aus der Luft auftreten. Dadurch bleiben Blushing-Effekte bei der flächigen Anwendung auch unter ungünstigen, das heisst das Blushing begünstigenden, Reaktionsbedingungen, nämlich bei tiefer Aushärtungstemperatur, beispielsweise im Bereich von 0 bis 10 °C, und hoher Luftfeuchtigkeit, weitgehend aus. Es ist deshalb möglich, weitgehend oder ganz auf den Zusatz von das Blushing vermindernden Zusätzen nach dem Stand der Technik, welche bei der Aushärtung nicht kovalent in der Zusammensetzung gebunden werden und als VOC ausgasen, wie insbesondere Benzylalkohol, zu verzichten. Die beschriebenen Epoxidharz-Zusammensetzungen **Z2** lassen sich somit besonders vorteilhaft auch in Innenräumen anwenden.

Die beschriebenen Epoxidharz-Zusammensetzungen **Z2** können verwendet werden als Vergussmassen, Dichtstoffe, Klebstoffe, Primer oder Schäume, sowie insbesondere für flächige Applikationen, insbesondere als Beläge, Beschichtungen, Anstriche, Lacke, Versiegelungen und Grundierungen, für Bau- und Industrieanwendungen, beispielsweise als Bodenbeläge und Bodenbeschichtungen für Innenräume wie Büros, Industriehallen, Turnhallen oder Kühlräume, oder im Aussenbereich für Balkone, Terrassen, Parkdecks, Brücken oder Dächer, sowie als Schutzbeschichtung für Beton oder Metalle, insbesondere als Schutzanstrich gegen Korrosion, sowie als Oberflächenversiegelung für solche Beläge.

Bei diesen Verwendungen kommen ihre ausgezeichneten Eigenschaften wie Wasserdichtigkeit, Korrosionsschutz, Haftung, Chemikalienbeständigkeit und/oder Härte und Zähigkeit, sowie insbesondere ihre hervorragenden Eigenschaften bezüglich flächiger Aushärtung zu weitgehend klaren, glänzenden und klebefreien Filmen ohne Blushing-bedingte Oberflächendefekte, zum Tragen.

### Beispiele

### 1. Beschreibung der Messmethoden

Der **Amingehalt**, das heisst der totale Gehalt an freien Aminogruppen und blockierten Aminogruppen (Iminogruppen) in den hergestellten Verbindungen, wurde titrimetrisch bestimmt (mit 0.1N HClO₄ in Eisessig, gegen Kristallviolett) und ist stets angegeben in mmol N/g.

**Infrarotspektren** wurden als unverdünnte Filme (falls nötig durch Lösen der Substanz in CH₂Cl₂ und Eindampfen des Lösemittels aufgebracht) auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹); der Zusatz sh weist auf eine als Schulter erscheinende Bande hin.

**¹H-NMR-Spektren** wurden auf einem Spektrometer des Typs Bruker DPX-300 bei 300.13 MHz gemessen; die chemischen Verschiebungen δ sind angegeben in ppm relativ zu Tetramethylsilan (TMS), Kopplungskonstanten J sind angegeben in Hz. Echte und Pseudo-Kopplungsmuster wurden nicht unterschieden.

Die **Viskositäten** wurden auf einem Kegel-Platten-Viskosimeter Rheotec RC30 (Kegeldurchmesser 50 mm, Kegelwinkel 1°, Kegelspitze-Platten-Abstand 0.05 mm, Scherrate 10-100 s⁻¹) gemessen.

### 2. Herstellung von Aldehyden der Formel (III)

### 2,2-Dimethyl-3-bis(2-methoxyethyl)amino-propanal

In einem Rundkolben wurden unter Stickstoffatmosphäre 31.3 g (0.37 mol) 36%iger wässriger Formaldehyd und 28.4 g (0.39 mol) Isobutyraldehyd vorgelegt. Unter gutem Rühren und Eiskühlung wurden aus einem Eintropftrichter langsam 50.0 g (0.37 mol) Bis(2-methoxyethyl)amin zugetropft, wobei darauf geachtet wurde, dass die Temperatur der Reaktionsmischung nicht über 20 °C anstieg. Nach erfolgter Zugabe liess man eine Stunde bei Raumtemperatur rühren. Die entstandene Reaktionsmischung wurde im Ölbad bei 100 °C während 18 Stunden unter Rückfluss gerührt, auf Raumtemperatur abgekühlt und die Phasen im Scheidetrichter getrennt. Die organische Phase wurde ohne weitere Aufbereitung im Vakuum fraktioniert. Das Produkt destillierte bei einer Kopftemperatur von 66 °C und einem Druck von 0.04 mbar.

### 2,2-Dimethyl-3-(N-morpholino)-propanal

Auf die gleiche Weise wie für 2,2-Dimethyl-3-bis(2-methoxyethyl)-amino-propanal beschrieben wurden 83.1 g (1.00 mol) 36%iger wässriger Formaldehyd, 75.0 g (1.04 mol) Isobutyraldehyd und 87.1 g (1.00 mol) Morpholin miteinander umgesetzt. Bei der Reinigung destillierte das Produkt bei einer Kopftemperatur von 97 °C und einem Druck von 14 mbar.

### 2,2-Dimethyl-3-dimethylamino-propanal

Auf die gleiche Weise wie für 2,2-Dimethyl-3-bis(2-methoxyethyl)-amino-propanal beschrieben wurden 83.1 g (1.00 mol) 36%iger wässriger Formaldehyd, 79.3 g (1.10 mol) Isobutyraldehyd und 112.7 g (1.00 mol) 40%iges wässriges Dimethylamin miteinander umgesetzt. Bei der Reinigung destillierte das Produkt bei einer Kopftemperatur von 94 °C und einem Druck von 16 mbar.

### 2,2-Dimethyl-3-(N-pyrrolidino)-propanal

Auf die gleiche Weise wie für 2,2-Dimethyl-3-bis(2-methoxyethyl)-amino-propanal beschrieben wurden 59.8 g (0.72 mol) 36%iger wässriger Formaldehyd, 53.8 g (0.75 mol) Isobutyraldehyd und 51.0 g (0.72 mol) Pyrrolidin miteinander umgesetzt. Bei der Reinigung destillierte das Produkt bei einer Kopftemperatur von 80 °C und einem Druck von 17 mbar.

### 2,2-Dimethyl-3-methylamino-propanal

In einem Rundkolben wurden unter Stickstoffatmosphäre 14.0 g (0.47 mol) Paraformaldehyd, 36.0 g (0.50 mol) Isobutyraldehyd und 24.0 g (0.36 mol) Methylamin-hydrochlorid vorgelegt. Die Reaktionsmischung wurde im Ölbad bei 100 °C während 4 Stunden unter Rückfluss gerührt, wobei eine homogene gelbliche Flüssigkeit entstand, welche beim Abkühlen auf Raumtemperatur erstarrte. Das Produkt wurde in 150 ml Wasser aufgenommen, mit Diethylether gewaschen und die wässrige Phase mit KOH neutralisiert. Dabei wurde ein gelblich-braunes Öl abgeschieden, welches in Diethylether aufgenommen wurde. Die etherische Lösung wurde mit Kaliumcarbonat getrocknet, am Vakuum vollständig eingeengt und der Rückstand im Vakuum fraktioniert. Das Produkt destillierte bei einer Kopftemperatur von 48 °C und einem Druck von 16 mbar.

### N,N'-Bis(2,2-dimethyl-3-oxopropyl)-piperazin

In einem Rundkolben wurden unter Stickstoffatmosphäre 166.8 g (2.00 mol) 36%iger wässriger Formaldehyd und 150.4 g (2.08 mol) Isobutyraldehyd vorgelegt. Unter gutem Rühren und Eiskühlung wurden aus einem Eintropftrichter langsam 86.1 g (1.00 mol) Piperazin zugetropft, wobei darauf geachtet wurde, dass die Temperatur der Reaktionsmischung nicht über 20 °C anstieg. Nach erfolgter Zugabe liess man eine Stunde bei Raumtemperatur rühren. Die entstandene dickflüssige Suspension wurde im Ölbad bei 120 °C während 18 Stunden unter Rückfluss gerührt. Die klare, dunkelorange Reaktionsmischung wurde auf Raumtemperatur abgekühlt, wobei sie durchkristallisierte. Die feste Masse wurde mit einem Stössel zerkleinert, in Wasser suspendiert und abgenutscht. Das Rohprodukt wurde aus Ethylacetat umkristallisiert, wobei blassgelbe, geruchlose Kristallnadeln erhalten wurden.

### 3. Herstellung von Polyaminen der Formel (I)

**verwendete Substanzen:**

| | |
|---|---|
| Jeffamine^{®} D-230 (Huntsman) | Polypropylenglykol-Diamin, mittleres Molekulargewicht ca. 240 g/mol, Amingehalt ca. 8.29 mmol N/g |
| N4-Amin (BASF) | N,N'-Bis(3-aminopropyl)ethylendiamin |
| TETA (Delamine) | Triethylentetramin (technisch), Amingehalt ca. 25.67 mmol N/g |
| TEPA (Delamine) | Tetraethylenpentamin (technisch), Amingehalt ca. 24.06 mmol N/g |

### Allgemeine Herstellvorschrift für die reduktive Alkylierung

In einem Rundkolben wurden ein Aldehyd und ein Amin unter Stickstoffatmosphäre in ausreichend Isopropanol gelöst. Die Lösung wurde während 30 Minuten bei Raumtemperatur gerührt und anschliessend bei einem Wasserstoff-Druck von 80 bar, einer Temperatur von 80 °C und einem Fluss von 3 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die Lösung im Vakuum bei 80 °C eingeengt.

### Beispiel 1:

Gemäss der allgemeinen Herstellvorschrift für die reduktive Alkylierung wurden 21.73 g 2,2-Dimethyl-3-bis(2-methoxyethyl)amino-propanal und 12.00 g Jeffamine^{®} D-230 umgesetzt. Ausbeute: 29.31 g eines klaren, farblosen Öls mit einer Viskosität von 220 mPa·s bei 20 °C und einem Amingehalt von 5.94 mmol N/g.
FT-IR: 3336 (N-H), 2962, 2925, 2868, 2812, 1724, 1457, 1372, 1301, 1274, 1195, 1115, 1015, 960, 927, 837, 787.

### Beispiel 2:

Gemäss der allgemeinen Herstellvorschrift für die reduktive Alkylierung wurden 34.25 g 2,2-Dimethyl-3-(N-morpholino)-propanal und 24.00 g Jeffamine^{®} D-230 umgesetzt. Ausbeute: 52.08 g eines klaren, hellgelben Öls mit einer Viskosität von 430 mPa·s bei 20 °C und einem Amingehalt von 6.79 mmol N/g.
FT-IR: 3334 (N-H), 2954, 2880, 2849, 2799, 1474, 1454, 1373, 1359, 1317, 1283, 1262, 1203, 1116, 1070, 1037, 1012, 932, 863, 804, 760.

### Beispiel 3:

Gemäss der allgemeinen Herstellvorschrift für die reduktive Alkylierung wurden 20.54 g 2,2-Dimethyl-3-(N-morpholino)-propanal und 10.45 g N4-Amin umgesetzt. Ausbeute: 27.5 g eines klaren, farblosen Öls mit einer Viskosität von 980 mPa·s bei 20 °C C und einem Amingehalt von 11.92 mmol N/g.
FT-IR: 3296 (N-H), 2947, 2914 sh, 2887, 2847, 2798, 2681 sh, 1977, 1454, 1394, 1373, 1351, 1317, 1282, 1263, 1202, 1135 sh, 1115, 1070, 1037, 1011, 932, 881 sh, 862, 804, 743.
¹H-NMR (CDCl₃, 300 K): δ 3.66 (*t*, *J* = 4.6, 8 H, OC*H*₂CH₂N), 2.71 (s, 4 H, NHC*H*₂C*H*₂NH), 2.66 und 2.62 (2×*t*, *J* = 6.0, 2×4 H, NHC*H*₂CH₂C*H*₂NH), 2.50 (*t*, *J* = 4.6, 8 H, OCH₂C*H*₂N), 2.40 (*s*, 4 H, NC*H*₂C(CH₃)₂), 2.16 (s, 4 H, NHC*H*₂C(CH₃)₂), 1.66 (*quint*, *J* = 6.9, 4 H, NHCH₂C*H*₂CH₂NH), 1.25 (br s, 4 H, NH), 0.87 (s, 12 H, C(CH₃)₂).

### Beispiel 4:

Gemäss der allgemeinen Herstellvorschrift für die reduktive Alkylierung wurden 34.24 g 2,2-Dimethyl-3-(N-morpholino)-propanal und 18.41 g TETA umgesetzt. Ausbeute: 41.5 g eines klaren, hellgelben Öls mit einer Viskosität von 900 mPa·s bei 20 °C und einem Amingehalt von 12.82 mmol N/g.
FT-IR: 3311 (N-H), 2947, 2911 sh, 2886, 2846, 2796, 2685 sh, 1454, 1393, 1372, 1357, 1317, 1282, 1264, 1202, 1133 sh, 1115, 1070, 1037, 1011, 932, 877 sh, 862, 804, 760.

### Beispiel 5:

Gemäss der allgemeinen Herstellvorschrift für die reduktive Alkylierung wurden 26.59 g 2,2-Dimethyl-3-(N-morpholino)-propanal und 14.0 g TEPA umgesetzt. Ausbeute: 34.95 g eines klaren, farblosen Öls mit einer Viskosität von 2800 mPa·s bei 20 °C und einem Amingehalt von 11.21 mmol N/g. FT-IR: 3308 (N-H), 2886, 2855, 2802, 2674, 1456, 1395, 1374, 1357, 1317, 1282, 1264, 1203, 1114, 1070, 1037, 1011, 932, 863, 804, 770.

### Beispiel 6:

Gemäss der allgemeinen Herstellvorschrift für die reduktive Alkylierung wurden 35.96 g 2,2-Dimethyl-3-(N-morpholino)-propanal und 17.03 g 1-Amino-3-aminomethyl-3,5,5-trimethylcyclohexan (= Isophorondiamin) umgesetzt. Ausbeute: 43.4 g eines klaren, farblosen Öls mit einer Viskosität von 8'800 mPa·s bei 20 °C und einem Amingehalt von 8.04 mmol N/g.
FT-IR: 3313 (N-H), 2949, 2890, 2847, 2799, 1652, 1456, 1375, 1360, 1317, 1282, 1263, 1202, 1116, 1070, 1037, 1011, 932, 863, 805, 745, 668.

### Beispiel 7:

Gemäss der allgemeinen Herstellvorschrift für die reduktive Alkylierung wurden 26.4 g 2,2-Dimethyl-3-(N-morpholino)-propanal und 10.0 g 1,3-Bis-(aminomethyl)-benzol (= meta-Xylylendiamin) umgesetzt. Ausbeute: 31.5 g eines klaren, farblosen Öls mit einer Viskosität von 950 mPa·s bei 20 °C und einem Amingehalt von 8.93 mmol N/g.
FT-IR: 3314 (N-H), 2951, 2888, 2848, 2799, 2685, 1977, 1935, 1607, 1590, 1454, 1394, 1374, 1357, 1316, 1282, 1263, 1202, 1116, 1070, 1037, 931, 862, 804, 780, 752, 700.

### Beispiel 8:

Gemäss der allgemeinen Herstellvorschrift für die reduktive Alkylierung wurden 53.94 g 2,2-Dimethyl-3-(N-morpholino)-propanal und 21.34 g 1,3-Bis-(aminomethyl)cyclohexan umgesetzt. Ausbeute: 65.2 g eines klaren, farblosen Öls mit einer Viskosität von 1'900 mPa·s bei 20 °C und einem Amingehalt von 9.08 mmol N/g.
FT-IR: 3315 (N-H), 2951, 2887, 2848, 2799, 1977, 1940, 1607, 1589, 1454, 1395, 1358, 1316, 1282, 1263, 1202, 1114, 1070, 1037, 931, 862, 804, 781, 702,668.

### Beispiel 9:

Gemäss der allgemeinen Herstellvorschrift für die reduktive Alkylierung wurden 35.96 g 2,2-Dimethyl-3-(N-morpholino)-propanal und 21.04 g Bis-(p-aminocyclohexyl)methan umgesetzt. Ausbeute: 49.5 g eines klaren, farblosen Öls mit einer Viskosität von 3'000 mPa·s bei 20 °C und einem Amingehalt von 8.83 mmol N/g.
FT-IR: 3305 (N-H), 2915, 2847, 2798, 1558, 1454, 1374, 1358, 1318, 1283, 1262, 1202, 1115, 1070, 1011, 932, 863, 804, 720, 668.

### Beispiel 10:

Gemäss der allgemeinen Herstellvorschrift für die reduktive Alkylierung wurden 35.96 g 2,2-Dimethyl-3-(N-morpholino)-propanal und 16.60 g einer Lösung von 70 Gewichts-% Hexamethylendiamin in Wasser umgesetzt. Ausbeute: 40.50 g eines klaren, farblosen Öls mit einer Viskosität von 400 mPa·s bei 20 °C und einem Amingehalt von 9.14 mmol N/g.
FT-IR: 3340 (N-H), 2967, 2929, 2809, 1652, 1465, 1404, 1377, 1340, 1306, 1160, 1112, 1070, 1037, 1010, 951, 862, 816.

### Beispiel 11:

Gemäss der allgemeinen Herstellvorschrift für die reduktive Alkylierung wurden 35.96 g 2,2-Dimethyl-3-(N-morpholino)-propanal und 12.82 g einer Mischung aus ca. 80 Gewichts-% 1,3-Diamino-4-methyl-cyclohexan und ca. 20 Gewichts-% 1,3-Diamino-2-methyl-cyclohexan umgesetzt. Ausbeute: 42.20 g eines klaren, leicht gelblichen Öls mit einer Viskosität von 3'700 mPa·s bei 20 °C und einem Amingehalt von 8.91 mmol N/g.
FT-IR: 3320 (N-H), 2949, 2848, 2799, 1734, 1668, 1455, 1374, 1358, 1318, 1282, 1261, 1203, 1140, 1115, 1070, 1036, 1011, 932, 863, 804, 729.

### Beispiel 12:

Gemäss der allgemeinen Herstellvorschrift für die reduktive Alkylierung wurden 35.9 g 2,2-Dimethyl-3-(N-morpholino)-propanal und 6.0 g Ethylendiamin umgesetzt. Ausbeute: 37.79 g einer weisslichen kristallinen Substanz mit einem Amingehalt von 10.5 mmol N/g.
FT-IR: 3318 (N-H), 2950, 2887, 2850, 2799, 2688, 1978, 1935, 1726, 1679, 1612, 1590, 1454, 1395, 1374, 1358, 1316, 1282, 1264, 1203, 1116, 1070, 1037, 1011, 932, 910, 863, 804, 780, 753.

### Beispiel 13:

Gemäss der allgemeinen Herstellvorschrift für die reduktive Alkylierung wurden 25.84 g 2,2-Dimethyl-3-dimethylamino-propanal und 24.00 g Jeffamine^{®} D-230 umgesetzt. Ausbeute: 45.50 g eines klaren, farblosen Öls mit einer Viskosität von 170 mPa·s bei 20 °C und einem Amingehalt von 8.5 mmol N/g.
FT-IR: 3327 (N-H), 2942, 2864, 2815, 2763, 1885, 1638, 1453, 1372, 1263, 1154, 1110, 1095, 1042, 908, 844, 788, 758.

### Beispiel 14:

Gemäss der allgemeinen Herstellvorschrift für die reduktive Alkylierung wurden 19.5 g 2,2-Dimethyl-3-dimethylamino-propanal und 13.05 g N4-Amin umgesetzt. Ausbeute: 25.2 g eines klaren, farblosen Öls mit einer Viskosität von 78 mPa·s bei 20 °C und und einem Amingehalt von 14.90 mmol N/g.
FT-IR: 3297 (N-H), 2939, 2840, 2813, 2763, 1453, 1374, 1359, 1317, 1263, 1120, 1042, 956, 899, 844, 741.
¹H-NMR (CDCl₃, 300 K): δ 2.71 (*s*, 4 H, NHC*H*₂C*H*₂NH), 2.66 und 2.64 (2×*t*, *J* = 6.8, 2×4 H, NHC*H*₂CH₂C*H*₂NH), 2.41 (*s*, 4 H, NC*H*₂C(CH₃)₂), 2.27 (*s*, 12 H, N(CH₃)₂), 2.14 (*s*, 4 H, NHC*H*₂C(CH₃)₂), 1.67 (*quint*, *J* = 6.9, 4 H, NHCH₂C*H*₂CH₂NH), 1.3 (br *s*, 4 H, NH), 0.88 (*s*, 12 H, C(CH₃)₂).

### Beispiel 15:

Gemäss der allgemeinen Herstellvorschrift für die reduktive Alkylierung wurden 13.87 g 2,2-Dimethyl-3-dimethylamino-propanal und 9.88 g TETA umgesetzt. Ausbeute: 20.30 g eines klaren, hellgelben Öls mit einer Viskosität von 54 mPa·s bei 20 °C und einem Amingehalt von 15.5 mmol N/g.
FT-IR: 3299 (N-H), 2939, 2841, 2812, 2763, 1738, 1667, 1454, 1378, 1360, 1334, 1263, 1122, 1042, 938, 896, 844, 754.

### Beispiel 16:

Gemäss der allgemeinen Herstellvorschrift für die reduktive Alkylierung wurden 67.83 g 2,2-Dimethyl-3-dimethylamino-propanal und 35.56 g 1,3-Bis-(aminomethyl)cyclohexan umgesetzt. Ausbeute: 87.8 g eines klaren, farblosen Öls mit einer Viskosität von 160 mPa·s bei 20 °C und und einem Amingehalt von 10.24 mmol N/g.
FT-IR: 3345 (N-H), 3217, 3034, 2911, 2841, 1912, 1833, 1728, 1603, 1491, 1459, 1374, 1312, 1161, 1126, 1043, 995, 952, 866, 777, 691.

### Beispiel 17:

Gemäss der allgemeinen Herstellvorschrift für die reduktive Alkylierung wurden 31.05 g 2,2-Dimethyl-3-(N-pyrrolidino)-propanal und 24.00 g Jeffamine^{®} D-230 umgesetzt. Ausbeute: 50.86 g eines klaren, farblosen Öls mit einer Viskosität von 200 mPa·s bei 20 °C und einem Amingehalt von 7.7 mmol N/g.
FT-IR: 3336 (N-H), 2962, 2870, 2780, 1652, 1472, 1372, 1340, 1306, 1285, 1240, 1108, 1030, 906, 874, 788.

### Beispiel 18:

Gemäss der allgemeinen Herstellvorschrift für die reduktive Alkylierung wurden 11.5 g frisch destilliertes 2,2-Dimethyl-3-methylamino-propanal und 12.00 g Jeffamine^{®} D-230 umgesetzt. Ausbeute: 20.2 g eines klaren, farblosen Öls mit einer Viskosität von 200 mPa·s bei 20 °C.
FT-IR: 3316 (N-H), 2961, 2867, 1679, 1467, 1451, 1372, 1345, 1300, 1277, 1242, 1149 sh, 1110, 1034, 1011, 908, 844, 815, 742, 681.

### Beispiel 19:

Gemäss der allgemeinen Herstellvorschrift für die reduktive Alkylierung wurden 34.98 g N,N'-Bis(2,2-dimethyl-3-oxopropyl)-piperazin und 42.58 g Isophorondiamin umgesetzt. Ausbeute: 69.00 g eines fast klaren, gelblichen Öls mit einer Viskosität von 29'100 mPa·s bei 20 °C und einem Amingehalt von 10.7 mmol N/g.
FT-IR: 3307 (N-H), 2946, 2893, 2833, 2800, 1580, 1558, 1459, 1376, 1362, 1318, 1278, 1150, 1118, 1067, 1016, 892, 831, 769.

### 4. Herstellung von Epoxidharz-Zusammensetzungen

**verwendete Substanzen:**

| | |
|---|---|
| Araldite^{®} GY 250 (Huntsman) | Bisphenol-A-Diglycidylether, EEW ca. 187.5 g/Eq |
| Araldite^{®} DY-E (Huntsman) | Monoglycidylether eines C₁₂- bis C₁₄-Alkohols, EEW ca. 290 g/Eq |
| Ancamine^{®} K 54 (Air Products) | 2,4,6-Tris-(dimethylaminomethyl)-phenol |

### Beispiele 20 bis 25

Für jedes Beispiel wurden die in der Tabelle 1 angegebenen Inhaltsstoffe in den angegebenen Mengen (in Gewichtsteilen) mittels eines Zentrifugalmischers (SpeedMixer™ DAC 150, FlackTek Inc.) vermischt. Mit den vermischten Zusammensetzungen wurde jeweils, sofern angegeben, die Viskosität 10 Minuten nach dem Vermischen bei 20 °C bestimmt. Weiterhin wurde jeweils ein Film in einer Schichtdicke von 500 µm auf eine Glasplatte aufgezogen und dieser bei 23 °C und 50 % relativer Feuchtigkeit (= Normklima, im Folgenden abgekürzt mit "NK") gelagert, beziehungsweise ausgehärtet. Nach 4 Wochen wurde der Aspekt der Filme beurteilt. Als "fehlerfrei" wurde ein Film bezeichnet, welcher klar war und eine harte, glänzende und klebefreie Oberfläche ohne Struktur aufwies. Als "Struktur" wird dabei jegliche Form von Zeichnung oder Muster auf der Oberfläche bezeichnet. Als "matt" wurde ein Film bezeichnet, welcher klar war und eine harte, klebefreie Oberfläche ohne Struktur aufwies, jedoch ohne Glanz. Weiterhin wurde die Königshärte (Pendelhärte nach König, gemessen nach DIN EN ISO 1522) der Filme nach 7 Tagen ("Königshärte (7d)") bzw. nach 14 Tagen ("Königshärte (14d)") bzw. nach 4 Wochen ("Königshärte (4w)") bestimmt.

Die Resultate sind in der Tabelle 1 angegeben.

**Tabelle 1: Zusammensetzung und Eigenschaften der Beispiele 20 bis 25. "Bsp." steht für "Beispiel" "n.b." steht für "nicht bestimmt"**

| **Beispiel** | | **20** | **21** | **22** | **23** | **24** | **25** |
|---|---|---|---|---|---|---|---|
| Araldite^{®} GY-250 | | 167.2 | 167.2 | 167.2 | 167.2 | 167.2 | 167.2 |
| Araldite^{®} DY-E | | 31.8 | 31.8 | 31.8 | 31.8 | 31.8 | 31.8 |
| Isophorondiamin | | - | - | 18.9 | - | 21.3 | - |
| Jeffamine^{®} D-230 | | - | - | - | - | - | 24.0 |
| Polyamin **Bsp. 3** | | 121.0 | - | - | - | - | - |
| Polyamin **Bsp. 5** | | - | 99.8 | 55.4 | - | - | - |
| Polyamin **Bsp. 14** | | - | - | - | 100.2 | - | - |
| Polyamin **Bsp. 18** | | - | - | - | - | 54.8 | - |
| Polyamin **Bsp. 19** | | - | - | - | - | - | 56.3 |
| Ancamine^{®} K 54 | | - | 6.0 | 5.6 | 6.0 | 5.6 | 5.6 |
| | | | | | | | |
| Viskosität (10') [Pa.s] | | n.b. | n.b. | n.b. | 0.56 | 0.50 | 1.24 |
| Königshärte [s] | (7d) | 35 | 108 | 126 | 85 | 87 | 188 |
| | (14d) | 92 | 140 | 182 | 86 | 139 | 197 |
| | (4w) | 154 | 160 | 197 | 86 | 148 | 205 |
| Aspekt | | fehlerfrei | fehlerfrei | leichte Struktur | fehlerfrei | matt | fehlerfrei |

Aus der Tabelle 1 ist ersichtlich, dass die Epoxidharz-Zusammensetzungen der Beispiele 20 bis 25 zu qualitativ hochwertigen, klaren Filmen aushärteten.

### 6. Herstellung von Isocyanatgruppen aufweisenden Zusammensetzungen

**verwendete Substanzen:**

| | |
|---|---|
| Jeffamine^{®} D-2000 (Huntsman) ("*D-2000*") | Polypropylenglykol-Diamin, mittleres Molekulargewicht ca. 2000 g/mol, Amingehalt ca. 0.98 mmol N/g |
| Jeffamine^{®} SD-2001 (Huntsman) ("*SD-2001*") | N, N'-Diisopropyl-polypropylenglykol-diamin, mittleres Molekulargewicht ca. 2000 g/mol, Amingehalt ca. 0.95 mmol N/g |
| Jeffamine^{®} T-5000 (Huntsman) ("*T-5000*") | Polypropylenglykol-Triamin, mittleres Molekulargewicht ca. 5000 g/mol, Amingehalt ca. 0.53 mmol N/g |
| Desmophen^{®} NH 1220 (Bayer) ("*NH 1220*") | Addukt aus 1,5-Diamino-2-methylpentan und Maleinsäurediethylester im Molverhältnis 1/2, Amingehalt ca. 4.35 mmol N/g |
| Desmophen^{®} NH 1420 (Bayer) ("*NH 1420*") | Addukt aus Bis-(4-aminocyclohexyl)-methan und Maleinsäurediethylester im Molverhältnis Amingehalt ca. 3.60 mmol N/g |
| Jefflink^{®} 754 (Huntsman) ("*Jefflink*") | N,N'-Diisopropyl-1-amino-3-aminomethyl-3,5,5-trimethylcyclohexan |
| Ethacure^{®} 100 (Albermale) ("*DETDA*") | Mischung aus ca. 80 Gew.-% 2,4-Diamino-3,5-diethyltoluol und ca. 20 Gew.-% 2,6-Di-amino-3,5-diethyltoluol |
| Ruetasolv^{®} DI (Rütgers) ("*Ruetasolv*") | Diisopropylnaphthalin |

### Beispiele 26 bis 31 und Vergleichsbeispiele 32 und 33:

Für jedes Beispiel wurden die in der Tabelle 2 angegebenen Inhaltsstoffe in den angegebenen Mengen (in Gewichtsteilen) mittels eines Zentrifugalmischers (SpeedMixer™ DAC 150, FlackTek Inc.) vermischt. Mit den vermischten Zusammensetzungen wurde jeweils ein Film mit einer Schichtdicke von ungefähr 2 mm gegossen und dieser während 10 Tagen im Normklima (23±1 °C und 50±5% relative Luftfeuchtigkeit) zur Aushärtung aufbewahrt. An den so ausgehärteten Filmen wurde der Aspekt beurteilt. Als "fehlerfrei" wurde ein Film bezeichnet, welcher klar und frei von Blasen war und eine klebfreie Oberfläche aufwies. Als "feine Blasen" wurde ein ansonsten fehlerfreier Film, der einige feine Bläschen enthielt, bezeichnet. Als "wenige Blasen" wurde ein ansonsten fehlerfreier Film bezeichnet, der wenige Blasen enthielt. An den so ausgehärteten Filmen wurden weiterhin die mechanische Eigenschaften geprüft, indem aus den Filmen jeweils einige Hanteln mit einer Länge von 75 mm, bei einer Steglänge von 30 mm und einer Stegbreite von 4 mm, ausgestanzt und gemäss DIN EN 53504 bei einer Zuggeschwindigkeit von 200 mm/min auf Zugfestigkeit (Bruchkraft), Bruchdehnung und E-Modul (bei 0.5-5.0 % Dehnung) geprüft wurden, sowie weiterhin die Shore-Härte (A/D) nach DIN 53505 bestimmt.

**Tabelle 2: Zusammensetzung und Eigenschaften der Beispiele 26 bis 31 und der Vergleichsbeispiele 32 und 33. * Polyamin des angegebenen Beispiels; "Vgl." steht für "Vergleich", "Zugf." für "Zugfestigkeit", "Br.dehn." für "Bruchdehnung" und "E-Mod." für "E-Modul".**

| **Beispiel** | **26** | **27** | **28** | **29** | **30** | **31** | **32 (Vgl.)** | **33 (Vgl.)** |
|---|---|---|---|---|---|---|---|---|
| Polymer ***P1*** | 100.0 | 100.0 | 100.0 | 100.0 | 100.0 | 100.0 | 100.0 | 100.0 |
| *D-2000* | 47.0 | 47.0 | 47.0 | - | 47.0 | 47.0 | 47.0 | 47.0 |
| *SD-2001* | - | - | - | 47.0 | - | - | - | - |
| *T-5000* | 10.0 | 10.0 | 10.0 | 10.0 | 10.0 | 10.0 | 10.0 | 10.0 |
| **Beispiel 6 *** | 68.1 | - | - | - | - | - | - | - |
| **Beispiel 8 *** | - | 62.1 | - | - | - | - | - | - |
| **Beispiel 9 *** | - | - | 69.8 | 69.8 | - | - | - | - |
| **Beispiel 10 *** | - | - | - | - | 56.5 | - | - | - |
| **Beispiel 16 *** | - | - | - | - | - | 52.2 | - | - |
| *NH 1220* | - | - | - | - | - | - | 65.0 | - |
| *Jefflink* | - | - | - | - | - | - | - | 35.9 |
| | | | | | | | | |
| Zugf. [MPa] | 6.0 | 1.3 | 5.4 | 2.8 | 2.2 | 2.9 | 3.4 | 2.1 |
| Br.dehn. [%] | 550 | 1040 | 450 | 370 | 500 | 850 | 930 | 500 |
| E-Mod. [MPa] | 88.5 | 8.2 | 92.9 | 44.6 | 10.5 | 25.4 | 3.8 | 28.6 |
| Shore-Härte | 38 D | 72 A | 40 D | 28 D | 20 D | 21 D | 14 D | 27 D |
| Aspekt | fehlerfrei | feine Blasen | fehlerfrei | fehlerfrei | wenige Blasen | fehlerfrei | feine Blasen | fehlerfrei |

### Polymer P1:

151.0 g Jeffamine^{®} SD-2001 (Huntsman) und 147.0 g Isophorondiisocyanat (Vestanat^{®} IPDI, Degussa) wurden bei Raumtemperatur zu einem NCO-terminierten Quasi-Prepolymer mit einem Gehalt an freien Isocyanatgruppen von 15.14 Gewichts-% und einer Viskosität bei 20 °C von 660 mPa·s umgesetzt.

Aus der Tabelle 2 ist ersichtlich, dass die erfindungsgemässen Beispiele 26 bis 31 zu qualitativ hochwertigen Filmen aushärteten. Die Beispiele 26 und 28 wiesen sehr hohe Härten auf, während das Beispiel 27 von sehr hoher Dehnbarkeit und Flexibilität war. Werden die ausgehärteten Filme enthaltend die Polyamine des Beispiels 8 und des Beispiels 16 miteinander verglichen, welche auf dem gleichen zugrunde liegenden primären Amin **PA**, aber auf verschiedenen Aldehyden **ALD** basieren (= Beispiele 27 und 31), so ergab das Polyamin des Beispiels 16 eine deutlich höhere Härte bei ebenfalls sehr hoher Dehnbarkeit des augehärteten Films.

### Beispiele 34 bis 40 und Vergleichsbeispiele 41 bis 43:

Für jedes Beispiel wurden die in der Tabelle 3 angegebenen Inhaltsstoffe in den angegebenen Mengen (in Gewichtsteilen) während 15 Sekunden mittels eines Zentrifugalmischers (SpeedMixer™ DAC 150, FlackTek Inc.) vermischt. Mit den vermischten Zusammensetzungen wurde jeweils die Zeit bis zur Gelierung der Mischung (kurz "Gelzeit") bestimmt, indem jeweils die Viskosität der vermischten Zusammensetzung bei 20 °C in regelmässigen Zeitintervallen gemessen und als Gelzeit die Zeitdauer unmittelbar nach dem Vermischen bis zum Überschreiten einer Viskosität von 100 Pa.s definiert war.

**Tabelle 3: Zusammensetzung und Gelzeit der Beispiele 34 bis 40 und der Vergleichsbeispiele 41 bis 43. "Vgl." steht für "Vergleich" * Polyamin des angegebenen Beispiels.**

| **Beispiel** | **34** | **35** | **36** | **37** | **38** | **39** | **40** | **41 (Vgl.)** | **42 (Vgl.)** | **43 (Vgl.)** |
|---|---|---|---|---|---|---|---|---|---|---|
| Polymer ***P1*** | 5.60 | 5.86 | 5.83 | 5.54 | 6.06 | 5.98 | 6.24 | 7.07 | 7.74 | 5.71 |
| *Ruetasolv* | 5.00 | 5.00 | 5.00 | 5.00 | 5.00 | 5.00 | 5.00 | 5.00 | 5.00 | 5.00 |
| **Beisp. 6 *** | 4.40 | - | - | - | - | - | - | - | - | - |
| **Beisp. 7 *** | - | 4.14 | - | - | - | - | - | - | - | - |
| **Beisp. 8 *** | - | - | 4.17 | - | - | - | - | - | - | - |
| **Beisp. 9 *** | - | - | - | 4.46 | - | - | - | - | - | - |
| **Beisp. 10 *** | - | - | - | - | 3.94 | - | - | - | - | - |
| **Beisp. 11 *** | - | - | - | - | - | 4.02 | - | - | - | - |
| **Beisp. 16 *** | - | - | - | - | - | - | 3.76 | - | - | - |
| *Jefflink* | - | - | - | - | - | - | - | 2.93 | - | - |
| *DETDA* | - | - | - | - | - | - | - | - | 2.26 | - |
| *NH 1220* | - | - | - | - | - | - | - | - | - | 4.29 |
| | | | | | | | | | | |
| Gelzeit | 23 min. | 9 min. | 6 min. | 70 s | <10s | 48 min. | 4 min. | 100 s | 110 s | 44 min. |

Aus der Tabelle 3 ist ersichtlich, dass die erfindungsgemässen Beispiele 34 bis 40 vergleichsweise lange Gelzeiten aufwiesen. Sie lagen, mit Ausnahme des Beispiels 38, im Bereich von gut einer Minute bis zu knapp einer Stunde, wobei das dem Polyamin der Formel (I) zugrunde liegende primäre Amin **PA** einen starken Einfluss auf die Reaktivität ausübte. Wird das Beispiel 34 mit dem Vergleichsbeispiel 41 verglichen, welchen beiden Isophorondiamin zugrunde lag, ist ersichtlich, dass das erfindungsgemässe Beispiel eine deutlich längere Gelzeit aufwies.

## Patentansprüche

1. Polyamin der Formel (I) 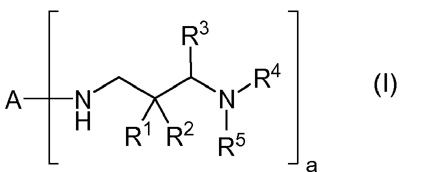 wobei
A für den Rest eines Amins nach Entfernung von a primären aliphatischen Aminogruppen steht;
a für eine ganze Zahl von 1 bis 6 steht, mit der Massgabe, dass im Fall von a = 1 der Rest A mindestens eine Reaktivgruppe ausgewählt aus der Gruppe bestehend aus primären Aminogruppen, sekundären Aminogruppen, Hydroxylgruppen und Mercaptogruppen aufweist;
R¹ und R² entweder
unabhängig voneinander jeweils für einen einwertigen Kohlenwasserstoffrest mit 1 bis 12 C-Atomen stehen,
oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
R³ für ein Wasserstoffatom oder eine Alkylgruppe oder eine Arylalkylgruppe oder eine Alkoxycarbonylgruppe steht;
und entweder
R⁴ für einen einwertigen aliphatischen, cycloaliphatischen oder arylaliphatischen Rest mit 1 bis 20 C-Atomen, welcher gegebenenfalls Heteroatome enthält, steht, und
R⁵ für Wasserstoffatom oder für einen einwertigen aliphatischen, cycloaliphatischen oder arylaliphatischen Rest mit 1 bis 20 C-Atomen, welcher gegebenenfalls Heteroatome enthält, steht,
oder
R⁴ und R⁵ zusammen für einen zweiwertigen aliphatischen Rest mit 3 bis 30 C-Atomen, der Teil eines, gegebenenfalls substituierten, heterocyclischen Rings mit 5 bis 8, bevorzugt 6, Ringatomen ist, wobei dieser Ring neben dem Stickstoffatom gegebenenfalls weitere Heteroatome enthält, stehen.

2. Polyamin der Formel (I) gemäss Anspruch 1, **dadurch gekennzeichnet, dass** R¹ und R² jeweils für einen Methylrest und/oder R³ für ein Wasserstoffatom stehen.

3. Polyamin der Formel (I) gemäss Anspruch 1 oder 2, **dadurch gekennzeichnet, dass** entweder R⁴ für Methyl, Ethyl, Propyl, Isopropyl, Butyl, 2-Ethylhexyl, Cyclohexyl, 2-Hydroxyethyl, 2-Hydroxypropyl oder Benzyl, und R⁵ für Wasserstoff oder Methyl, Ethyl, Propyl, Isopropyl, Butyl, 2-Ethylhexyl, Cyclohexyl, 2-Hydroxyethyl, 2-Hydroxypropyl oder Benzyl stehen, oder R⁴ und R⁵ zusammen - unter Einbezug des Stickstoffatoms - einen Ring bilden, insbesondere einen Pyrrolidin-, Piperidin-, Morpholin- oder N-Alkylpiperazin-Ring, wobei dieser Ring oder die Alkylgruppe gegebenenfalls substituiert sind.

4. Verfahren zur Herstellung eines Polyamins der Formel (I) gemäss einem der Ansprüche 1 bis 3, **dadurch gekennzeichnet, dass** mindestens ein Amin **PA** der Formel (II) mit mindestens einem Aldehyd **ALD** der Formel (III) reduktiv alkyliert wird.

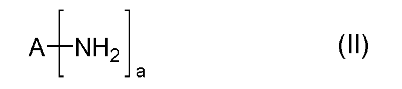

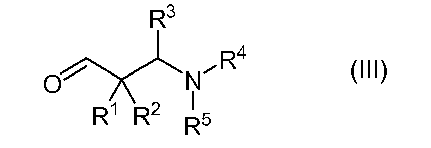

5. Verfahren zur Herstellung eines Polyamins der Formel (I) gemäss Anspruch 4, **dadurch gekennzeichnet, dass** der Prozess so geführt wird, dass mindestens ein Amin **PA** der Formel (II) mit mindestens einem Aldehyd **ALD** der Formel (III) zu einem Imin kondensiert und dieses anschliessend hydriert wird.

6. Verfahren gemäss Anspruch 4 oder 5, **dadurch gekennzeichnet, dass** das Amin **PA** der Formel (II) ausgewählt ist aus der Gruppe bestehend aus 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,12-Dodecandiamin, 1,4-Diaminocyclohexan, Bis-(4-aminocyclohexyl)-methan (H₁₂-MDA), Bis-(4-amino-3-methylcyclohexyl)-methan, 1-Amino-3-aminomethyl-3,5,5-trimethylcyclohexan (= Isophorondiamin oder IPDA), 1,3-Bis-(aminomethyl)cyclohexan, 2,5(2,6)-Bis-(aminomethyl)-bicyclo[2.2.1]-heptan (NBDA), 3(4),8(9)-Bis-(aminomethyl)-tricyclo[5.2.1.0^{2,6}]decan, 1,3-Xylylendiamin, Bis-hexamethylentriamin (BHMT), Diethylentriamin (DETA), Triethylentetramin (TETA), Tetraethylenpentamin (TEPA), Pentaethylenhexamin (PEHA), Polyethylenpolyamin mit 5 bis 7 Ethylenamin-Einheiten (sogenanntes "higher ethylenepolyamine", HEPA), Dipropylentriamin (DPTA), N-(2-Aminoethyl)-1,3-propandiamin (N3-Amin), N,N'-Bis(3-aminopropyl)ethylendiamin (N4-Amin) und Polyoxyalkylen-Diamine und Polyoxyalkylen-Triamine, insbesondere mit einem Molekulargewicht von 200 bis 6000 g/mol.

7. Verfahren gemäss Anspruch 4 oder 5, **dadurch gekennzeichnet, dass** der Aldehyd **ALD** der Formel (III) ausgewählt ist aus der Gruppe bestehend aus 2,2-Dimethyl-3-methylamino-propanal, 2,2-Dimethyl-3-dimethylamino-propanal, 2,2-Dimethyl-3-ethylamino-propanal, 2,2-Dimethyl-3-diethylamino-propanal, 2,2-Dimethyl-3-butylamino-propanal, 2,2-Dimethyl-3-dibutylamino-propanal, 2,2-Dimethyl-3-hexylamino-propanal, 2,2-Dimethyl-3-(2-ethylhexyl)amino-propanal, 2,2-Dimethyl-3-dodecylamino-propanal, 2,2-Dimethyl-3-(N-pyrrolidino)-propanal, 2,2-Dimethyl-3-(N-piperidino)-propanal, 2,2-Dimethyl-3-(N-morpholino)-propanal, 2,2-Dimethyl-3-(N-(2,6-dimethyl)morpholino)-propanal, 2,2-Dimethyl-3-benzylamino-propanal, 2,2-Dimethyl-3-(N-benzylmethylamino)-propanal, 2,2-Dimethyl-3-(N-benzylisopropylamino)-propanal, 2,2-Dimethyl-3-cyclohexylamino-propanal, 2,2-Dimethyl-3-(N-cyclohexylmethylamino)-propanal und N,N'-Bis(2,2-dimethyl-3-oxopropyl)-piperazin.

8. Addukt **AD**, erhalten aus der Umsetzung von mindestens einem Polyamin der Formel (I) gemäss einem der Ansprüche 1 bis 3 mit mindestens einer Verbindung **VB**, die mindestens eine, bevorzugt mindestens zwei, Reaktivgruppen **RG** trägt, wobei die Reaktivgruppen **RG** ausgewählt sind aus der Gruppe bestehend aus Isocyanat-, Isothiocyanat-, Cyclocarbonat-, Epoxid-, Episulfid-, Aziridin-, Acryl-, Methacryl-, 1-Ethinylcarbonyl-, 1-Propinylcarbonyl-, Maleimid-, Citraconimid-, Vinyl-, Isopropenyl- und Allyl-Gruppen.

9. Addukt **AD** gemäss Anspruch 8, **dadurch gekennzeichnet, dass** dieses ein mindestens zwei Aminogruppen der Formel (VIII) aufweisendes Addukt **AD1** darstellt.

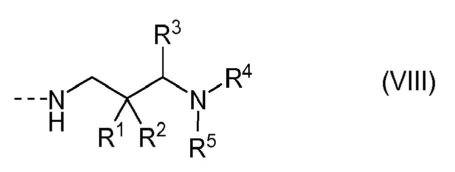

10. Addukt **AD** gemäss Anspruch 8, **dadurch gekennzeichnet, dass** dieses ein mindestens zwei Isocyanatgruppen aufweisendes Addukt **AD2** darstellt, wobei als Verbindung **VB** ein Polyisocyanat verwendet wurde.

11. Isocyanatgruppen aufweisende Zusammensetzung **Z1**, enthaltend
a) mindestens ein Polyisocyanat, und
b) mindestens eine Härter-Verbindung **HV**, welche mindestens zwei Reaktivgruppen, ausgewählt aus der Gruppe bestehend aus primären Aminogruppen, sekundären Aminogruppen, Hydroxylgruppen und Mercaptogruppen, aufweist;
mit der Massgabe, dass das Polyisocyanat und/oder die Härter-Verbindung **HV** eine Verbindung ausgewählt aus der Gruppe bestehend aus den Polyaminen der Formel (I) gemäss einem der Ansprüche 1 bis 3, den Addukten **AD1** gemäss Anspruch 9 und den Addukten **AD2** gemäss Anspruch 10 ist.

12. Ausgehärtete Zusammensetzung erhalten aus der Reaktion von mindestens einem Polyisocyanat mit mindestens einer Härter-Verbindung **HV** einer Isocyanatgruppen aufweisenden Zusammensetzung **Z1** gemäss Anspruch 11.

13. Epoxidharz-Zusammensetzungen **Z2**, enthaltend
a) mindestens ein Epoxidharz, und
b) mindestens ein Polyamin der Formel (I) gemäss einem der Ansprüche 1 bis 3.

14. Ausgehärtete Zusammensetzung erhalten aus der Reaktion von mindestens einem Epoxidharz mit mindestens einem Polyamin der Formel (I) einer Epoxidharz-Zusammensetzung **Z2** gemäss Anspruch 13.

15. Verwendung eines Polyamins der Formel (I) gemäss einem der Ansprüche 1 bis 3 oder eines Addukts **AD** gemäss einem der Ansprüche 8 bis 10 in Reinigungsmitteln, Treibstoffen, Schmierstoffen, Asphalten, Gummiartikeln, Pharmazeutika, Pflanzenschutzmitteln, Mahlhilfsmitteln, Sequestriermitteln, Erdölförderungshilfsmitteln, Papierchemikalien, Faserverbundwerkstoffen (Composites), Vergussmassen, Dichtstoffen, Klebstoffen, Belägen, Beschichtungen, Anstrichen, Lacken, Versiegelungen, Grundierungen, Primern, Schäumen, Formblöcken, Elastomeren, Fasern, Folien und Membranen.
